(19) Europäisches Patentamt
European Patent Office
Office européen des brevets

(11) Publication number: **0 029 303**
**B1**

(12) **EUROPEAN PATENT SPECIFICATION**

(45) Date of publication of patent specification: **30.01.85**

(21) Application number: **80303729.0**

(22) Date of filing: **22.10.80**

(51) Int. Cl.$^4$: **C 07 D 249/14,**
C 07 D 405/12,
C 07 D 409/12, A 61 K 31/41
// (C07D405/12, 307/52, 249/14),(C07D409/12, 333/20, 249/14)

# (54) 1,2,4-Triazole derivatives, processes for their production and pharmaceutical compositions containing them.

(30) Priority: **22.10.79 GB 7936545**
**22.10.79 GB 7936546**
**27.08.80 GB 8027740**

(43) Date of publication of application:
**27.05.81 Bulletin 81/21**

(45) Publication of the grant of the patent:
**30.01.85 Bulletin 85/05**

(84) Designated Contracting States:
**BE CH DE FR GB IT LI NL SE**

(56) References cited:
AT-B- 342 587
CH-A- 614 433
DE-A-2 734 070
DE-A-2 821 409
DE-A-2 821 410
US-A-3 686 201
US-A-3 813 400

(73) Proprietor: **GLAXO GROUP LIMITED**
**Clarges House 6-12 Clarges Street**
**London W1Y 8DH (GB)**

(72) Inventor: **Bradshaw, John**
**22 Whiteley Close**
**Dane End, Ware, Herts. (GB)**
Inventor: **Clitherow, John, Watson**
**54 Gilders**
**Sawbridgeworth, Herts (GB)**
Inventor: **Bays, David Edmund**
**9 Windmill Field**
**Ware, Herts (GB)**
Inventor: **Hayes, Roger**
**5 Sandringham Road**
**Potters Bar Herts (GB)**
Inventor: **MacKinnon, John Wilson Macfarlane**
**31 Trapstyle Road**
**Ware, Herts (GB)**

(74) Representative: **Marchant, James Ian et al**
**Elkington and Fife High Holborn House 52/54**
**High Holborn**
**London WC1V 6SH (GB)**

**EP 0 029 303 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to novel heterocyclic derivatives having action on histamine receptors, to processes for the preparation thereof, to pharmaceutical compositions containing them and to their use in therapeutics.

German Offenlegungsschrift No. 27 34 070 discloses aminoalkyl furan derivatives of the general formula

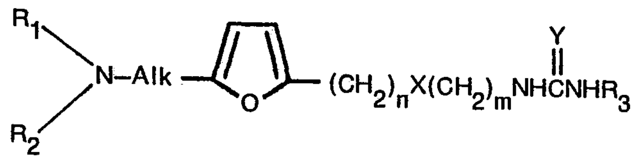

(A)

and physiologically acceptable salts, N-oxides and hydrates thereof.

German Offenlegungschrift No. 28 21 409 discloses aminoalkyl thiophene derivatives of the general formula

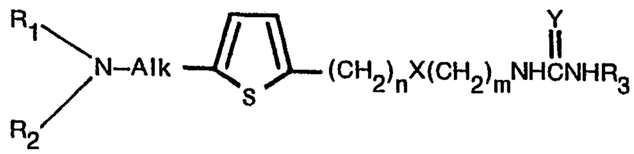

(B)

and physiologically acceptable salts, hydrates and bioprecursors thereof.

German Offenlegungsschrift No. 28 21 410 discloses aminoalkylbenzene derivatives of the general formula

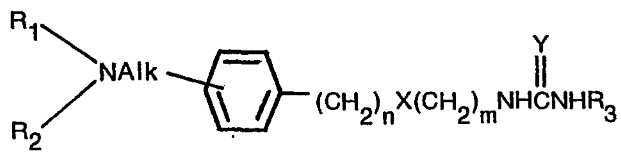

(C)

and physiologically acceptable salts, hydrates, N-oxides and bioprecursors thereof in which the substituents on the benzene ring can be ortho, meta or para to one another.

The compounds of formulae (A), (B) and (C) above have activity as selective histamine $H_2$-antagonists. In formulae (A), (B) and (C) above:

$R_1$ and $R_2$, which may be the same or different, each represent hydrogen, lower alkyl, cycloalkyl, aralkyl or lower alkenyl, or lower alkyl interrupted by an oxygen atom or the group

$$-\overset{|}{N}R_4$$

where $R_4$ is hydrogen or lower alkyl; or $R_1$ and $R_2$ together with the nitrogen atom to which they are attached, form a heterocyclic ring which contain other heterofunctions selected from —O— and

$$-\overset{|}{N}R_4;$$

$R_3$ represents hydrogen, lower alkyl, lower alkenyl or alkoxyalkyl;
X represents $-CH_2-$, —O— or —S—;
Y represents =S, =O, $=NR_5$ or $=CHR_6$;
Alk represents a straight or branched alkylene chain of 1 to 6 carbon atoms;
$R_5$ represents hydrogen, nitro, cyano, lower alkyl aryl, alkylsulphonyl or arylsulphonyl;
$R_6$ represents nitro, arylsulphonyl or alkylsulphonyl;
m is an integer from 2 to 4; and
n is zero, 1 or 2, with the proviso that in formulae (A) and (B) n can only represent zero if X represents S or $CH_2$.

Certain novel heterocyclic derivatives having a structure different from the compounds referred to above, have now been found which have potent activity as $H_2$-antagonists. These compounds, which are more particularly described below, for example show inhibition of the secretion of gastric acid when this is stimulated via histamine receptors (Ash and Schild, Brit. J. Pharmacol. Chemother, 1966, *27*, 427). Their ability to do so can be demonstrated in the perfused rat stomach using the method described in German Offenlegungsschrift No. 2,734,070, modified by the use of sodium pentobarbitone (50 mg/kg) as anaesthetic instead of urethane, and in conscious dogs equipped with Heidenhain pouches using the method described by Black *et al*, Nature 1972 *236*, 385. Furthermore the compounds antagonise the effect of histamine on the contraction frequency of isolated guinea pig right

atrium but do not modify histamine induced contractions of isolated gastro-intestinal smooth muscle which are mediated via $H_1$-receptors.

Compounds with histamine $H_2$-blocking activity may be used in the treatment of conditions where there is an advantage in lowering gastric acidity particularly in gastric and peptic ulceration, as a prophylactic measure in surgical procedures, and in the treatment of allergic and inflammatory conditions where histamine is a known mediator. Thus they may be used for example, either alone, or in combination with other active ingredients in the treatment of allergic and inflammatory conditions of the skin.

The present invention provides compounds of the general formula (I)

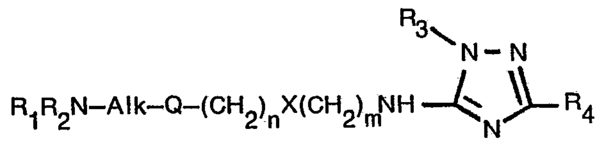

(I)

and physiologically acceptable salts and hydrates thereof, in which

$R_1$ represents a heteroaralkyl group made up of a heterocyclic part which is a furyl, thienyl, pyrrolyl, pyridinyl, pyrimidinyl, triazinyl, oxazolyl, triazolyl, thiazolyl, isoquinolinyl, quinolinyl or indolyl ring which ring is unsubstituted or is substituted by a substituent selected from $C_{1-3}$ alkyl, $C_{1-3}$ alkoxy, hydroxy, hydroxy $C_{1-4}$ alkyl, amino $C_{1-4}$ alkyl, $C_{1-4}$ alkylamino $C_{1-4}$ alkyl, di-$C_{1-4}$ alkylamino $C_{1-4}$ alkyl and halogen, and which ring is linked to the alkyl part through carbon, and an alkyl part which is a straight or branched $C_{1-4}$ alkyl chain, or

$R_1$ represents a $C_{11-16}$ straight or branched saturated or unsaturated alkylene chain optionally substituted by a hydroxy group or $R_1$ represents a $C_{1-16}$ straight or branched saturated or unsaturated alkylene chain substituted by an ester group $CO_2R_5$ or an amide group $CONH_2$, where $R_5$ is a $C_{1-3}$ alkyl group, $R_1$ represents a $C_1$—$C_{16}$ straight or branched saturated or unsaturated alkylene chain interrupted by an oxygen atom, a sulphur atom, a sulphoxide group, a sulphone group, an amide group (—CONH— or —NHCO—) or an ester group (—CO—O— or —O—CO), with the provisos that

$R_1$ does not represent a $C_{1-6}$ alkyl group substituted by a $C_{1-6}$ alkoxy group and that when the alkylene chain is interrupted by an oxygen or sulphur atom or a sulphoxide amide (CONH) or ester (COO) group there must be at least two carbon atoms between that group and the nitgrogen atom to which $R_1$ is attached;

$R_2$ represents hydrogen or a $C_{1-4}$ alkyl group;

Alk represents a straight or branched alkylene chain of 1 to 6 carbon atoms, preferably 1 to 4 carbon atoms;

Q represents a furan or thiophen ring in which incorporation into the rest of the molecule is through bonds at the 2- and 5-positions, the furan ring optionally bearing a further substituent $R_6$ adjacent to the group $R_1R_2NAlk$-, or Q represents a benzene ring in which incorporation into the rest of the molecule is through bonds at the 1- and 3- or 1- and 4-positions;

$R_6$ represents halogen, or $C_{1-4}$ alkyl which may be substituted by hydroxy or $C_{1-4}$ alkoxy;

X represents —$CH_2$—, —O—, —S—, or —NH—;

n represents zero, 1 or 2;

m represents 2, 3 or 4;

$R_3$ represents hydrogen, $C_{1-6}$ alkyl, $C_{3-6}$ alkenyl, or $C_{2-6}$ alkyl substituted by hydroxy or $C_{1-6}$ alkoxy; and

$R_4$ represents hydrogen, $C_{1-6}$ alkyl, $C_{3-6}$ alkenyl, phenyl $C_{1-3}$ alkyl, hydroxy $C_{1-6}$ alkyl, $C_{1-6}$ alkanoyloxy $C_{1-6}$ alkyl, $C_{1-6}$ alkoxy $C_{1-6}$ alkyl, amino $C_{1-6}$ alkyl, $C_{1-6}$ alkylamino $C_{1-6}$ alkyl, di-$C_{1-6}$ alkylamino $C_{1-6}$ alkyl, hydroxy or $C_{1-6}$ alkoxy or the group $NR_7R_8$ where

$R_7$ represents hydrogen, $C_{1-6}$ alkyl, $C_{1-6}$ alkyl substituted by hydroxy or $C_{1-3}$ alkoxy, $C_{3-6}$ alkenyl, phenyl $C_{1-3}$ alkyl or heteroaryl $C_{1-6}$ alkyl and $R_8$ represents any of the groups defined for $R_7$ or may represent the group $COR_9$ where $R_9$ represents hydrogen, $C_{1-6}$ alkyl, phenyl optionally substituted by a $C_{1-3}$ alkyl or $C_{1-3}$ alkoxy group, $C_{1-6}$ alkoxy, heteroaryl or heteroaryl $C_{1-6}$ alkyl or $R_8$ represents the group $SO_2R_{10}$ where $R_{10}$ represents $C_{1-6}$ alkyl or phenyl optionally substituted by a $C_{1-3}$ alkyl or $C_{1-3}$ alkoxy group, or $R_8$ represents the group

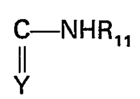

where Y is oxygen or sulphur and

$R_{11}$ represents hydrogen, $C_{1-6}$ alkyl, $C_{3-8}$ cycloalkyl or phenyl optionally substituted by a $C_{1-3}$ alkyl or $C_{1-3}$ alkoxy group, or

$R_7$ and $R_8$ taken together may represent the group $=CR_{12}R_{13}$ where $R_{12}$ represents phenyl or heteroaryl and $R_{13}$ represents hydrogen or $C_{1-6}$ alkyl with the provisos that (1) when X is —NH— then

n is zero, and (2) in the definition of the group $R_4$ the term "heteroaryl" means furyl, pyridyl, thiazolyl or thienyl.

The term "alkyl" as a group or part of a group means that the group is straight or branched. In the case where the alkyl groups have 1 to 6 carbon atoms they have preferably 1 to 4 carbon atoms and can be for example methyl or ethyl. Examples of alkanoyloxyalkyl groups include acetoxymethyl and formyloxymethyl. Examples of aroyloxyalkyl groups and aralkanoyloxyalkyl groups include benzyloxymethyl and phenylacetoxymethyl. The term "unsaturated" in relation to the alkylene chain within $R_1$ means that the chain contains at least one —C=C— or —C≡C— grouping.

In the term "heteroaralkyl" as applied to the group $R_1$ examples of the alkylene portion are methylene, ethylene and propylene groupings. When $R_1$ represents an optionally substituted alkylene chain this may for example be saturated and contain up to 16 carbon atoms, or be substituted by or interrupted by an optional amide or ester grouping.

The invention includes the compounds of formula (I) in the form of physiologically acceptable salts with inorganic and organic acids. Particularly useful salts include hydrochlorides, hydrobromides, sulphates, methanesulphonates, acetates, maleates, succinates, citrates, tartrates, benzoates and fumarates. The compounds of formula (I) and their salts may also form hydrates, which hydrates are also to be considered as part of the invention. The compounds of formula (I) can exhibit tautomerism and the formula is intended to cover all tautomers. Where optical isomers may exist the formula is intended to cover all diastereoisomers and optical enantiomers.

The compounds according to the invention, preferably in the form of a salt, may be formulated for administration in any convenient way and the invention includes within its scope pharmaceutical compositions containing at least one compound according to the invention adapted for use in human or veterinary medicine. Such compositions may be formulated in a conventional manner using one or more pharmaceutically acceptable carriers or excipients. Such compositions may also contain if required other active ingredients, e.g. $H_1$-antagonists.

Thus the compounds according to the invention may be formulated for oral, buccal, topical, parenteral or rectal administration. Oral administration is preferred.

For oral administration, the pharmaceutical composition may take the form of for example, tablets, capsules, powders, solutions, syrups or suspensions prepared by conventional means with acceptable excipients. For buccal administration the composition may take the form of tablets or lozenges formulated in conventional manner.

The compounds of the invention may be formulated for parenteral administration by bolus injection or continuous infusion. Formulations for injection may be presented in unit dosage form in ampoules, or in multi-dose containers, with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for reconstitution with a suitable vehicle, e.g. sterile pyrogen-free water before use.

The compounds of the invention may also be formulated in rectal compositions such as suppositories or retention enemas, e.g. containing conventional suppository bases such as cocoa butter or other glyceride.

For topical application, the compounds of the invention may be formulated as ointments, creams, gels, lotions, powders or sprays in conventional manner.

For internal administration a convenient daily dosage regime of the compounds according to the invention would be 1 to 4 doses to the total of some 5 mg to 2 g per day, preferably 5 to 500 mg per day, dependent upon the condition of the patient. Examples of suitable meanings for the groups $R_1$, $R_2$, $R_3$ and $R_4$ are as follows:—

$R_1$: a heteroaralkyl group in which the heterocyclic portion is a furyl, thienyl, pyrrolyl, pyridinyl, pyrimidinyl, triazinyl, oxazolyl, triazolyl, thiazolyl, isoquinolinyl, quinolinyl or indolyl ring, which ring is unsubstituted or substituted by a substituent selected from $C_{1-3}$ alkyl, $C_{1-3}$ alkoxy, hydroxy, hydroxy $C_{1-4}$ alkyl, amino $C_{1-4}$ alkyl, $C_{1-4}$ alkylamino $C_{1-4}$ alkyl, di-$C_{1-4}$ alkylamino $C_{1-4}$ alkyl and halogen, and the alkylene portion is a methylene, ethylene or propylene grouping; or an alkylene chain which is saturated and contains up to 16 carbon atoms or is substituted by or interrupted by an amide or ester grouping;

$R_2$: hydrogen or a methyl or ethyl group;

$R_3$: hydrogen, $C_{1-4}$ alkyl (e.g. methyl, ethyl or propyl), or hydroxy $C_{2-4}$ alkyl (e.g. 2-hydroxyethyl);

$R_4$: hydrogen, hydroxy, $C_{1-4}$ alkyl (e.g. methyl, ethyl isobutyl), hydroxy $C_{1-4}$ alkyl (e.g. hydroxymethyl, 2-hydroxyethyl or 1-hydroxy-1-methylethyl), $C_{1-3}$ alkoxy $C_{1-4}$ alkyl (e.g. methoxymethyl or methoxyethyl), phenyl $C_{1-3}$ alkyl (e.g. benzyl or phenethyl), $C_{2-4}$ alkanoyloxy $C_{1-4}$ alkyl (e.g. acetoxymethyl), amino $C_{1-4}$ alkyl (e.g. aminomethyl), amino, $C_{1-4}$ alkylamino (e.g. methylamino or ethylamino) or di-$C_{1-4}$ alkylamino (e.g. dimethylamino, diethylamino or dipropylamino), phenyl $C_{1-3}$ alkylamino (e.g. benzylamino), or a heteroaryl $C_{1-3}$ alkylamino group where the heteroaryl ring is furyl, pyridyl, thiazolyl or thienyl (e.g. 3- or 4-pyridylmethyl); or the group $NHCOR_9$ where $R_9$ represents hydrogen, $C_{1-3}$ alkyl (e.g. methyl or ethyl), $C_{1-3}$ alkoxy (e.g. methoxy or ethoxy), furyl,

pyridyl, thiazolyl, thienyl, or phenyl optionally substituted by a $C_{1-3}$ alkyl (e.g. methyl) or $C_{1-3}$ alkoxy (e.g. methoxy) group; or the group $NHSO_2R_{10}$ where $R_{10}$ represents $C_{1-3}$ alkyl (e.g. methyl), or phenyl optionally substituted by a $C_{1-3}$ alkyl (e.g. methyl) or $C_{1-3}$ alkoxy (e.g. methoxy) group; or the group $NHCONHR_{11}$ where $R_{11}$ is $C_{1-3}$ alkyl (e.g. methyl), $C_{5-7}$ cycloalkyl (e.g. cyclohexyl), or phenyl optionally substituted by a $C_{1-3}$ alkyl (e.g. methyl) or $C_{1-3}$ alkoxy (e.g. methoxy) group; or the group $N{=}CHR_{12}$ where $R_{12}$ is a phenyl or pyridyl (e.g. 3- or 4-pyridyl) group. The group Alk may be for example the group $(CH_2)_p$ where p is 1, 2 or 3.

Q may be for example a benzene ring incorporated into the rest of the molecule through bonds at the 1- and 3- or 1- and 4-positions, with n as zero, X as oxygen and m as 3 or 4; or Q may be a furan or thiophene ring with n as 1, X as sulphur and m as 2, with the furan ring optionally containing a further substituent $R_6$ where $R_6$ is bromine, $C_{1-3}$ alkyl (e.g. methyl, ethyl or isopropyl), or $C_{1-3}$ alkoxy-$C_{1-3}$ alkyl (e.g. methoxymethyl); or Q may be a furan ring with n as 1, X as $CH_2$ and m as 2.

In particular the groups $R_1$, $R_2$, $R_3$ and $R_4$ may have meanings as follows:

$R_1$: a heteroarylmethyl group where the heteroaryl ring is furyl, thienyl, pyrrolyl or pyridinyl linked to the methyl portion via a carbon atom; or a $C_{11-16}$ straight or branched saturated alkylene chain; or a $C_{1-16}$ straight or branched saturated alkylene chain interrupted by an amide (—NHCO—) group;
$R_2$: hydrogen or a methyl or ethyl group;
$R_3$: hydrogen, methyl, ethyl or 2-hydroxyethyl;
$R_4$: hydroxy, phenyl $C_{1-3}$ alkyl (e.g. benzyl); $C_{1-4}$ alkyl substituted by hydroxy, $C_{1-3}$ alkoxy, $C_{2-4}$ alkanoyloxy or amino (e.g. hydroxymethyl, 2-hydroxyethyl, acetoxymethyl or aminomethyl); amino, di $C_{1-4}$ alkylamino (e.g. diethylamino); $NHCOR_9$ where $R_9$ represents hydrogen, $C_{1-3}$ alkyl (e.g. methyl), $C_{1-3}$ alkoxy (e.g. ethoxy) or phenyl; $NHCONHR_{11}$ where $R_{11}$ represents phenyl, or $N{=}CHR_{12}$ where $R_{12}$ is phenyl or pyridyl (e.g. 4-pyridyl).

Alk is particularly a methylene or ethylene group, more particularly methylene.

Q is particularly a benzene ring incorporated into the rest of the molecule through bonds at the 1- and 3-positions, with n as zero, X as oxygen and m as 3 or 4; or a furan ring optionally containing a further substituent $R_6$ where $R_6$ is $C_{1-4}$ alkyl (e.g. methyl), with n as 1, X as sulphur and m as 2.

Preferably Q is a benzene ring in which incorporation into the rest of the molecule is through bonds at the 1- and 3-positions, n is zero, X is oxygen and m is 3 or 4. $R_1$ is preferably a heteroaralkyl group in which the alkyl portion is a methylene group.

A preferred group of compounds of formula (I) are those of formula (II)

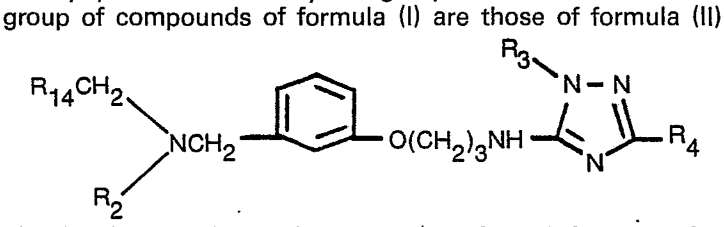

(II)

where $R_{14}$ is a furyl, thienyl, pyrrolyl or pyridinyl ring (e.g. 2-furyl, 2-thienyl, 2-pyrrolyl), $R_2$ is hydrogen or methyl; $R_3$ is hydrogen or methyl; and $R_4$ is an amino, hydroxy $C_{1-6}$ alkyl, $C_{1-6}$ alkanoyloxy $C_{1-6}$ alkyl, $C_{1-6}$ alkoxy $C_{1-6}$ alkyl, amino $C_{1-6}$ alkyl, benzyl, formamido, $C_{1-6}$ alkoxycarbonylamino, $C_{2-7}$ alkanoylamino, hydroxy, aroylamino (where the aryl group is phenyl optionally substituted by a $C_{1-3}$ alkyl or $C_{1-3}$ alkoxy group), phenylcarbonylamino, or pyridylmethylenamino group.

Particularly preferred compounds are:

1-methyl-$N^5$-[3-[3-[(dodecylamino)methyl]phenoxy]propyl]-1H-1,2,4-triazole-3,5-diamine
N-butyl-[3-[3-[(1-methyl-3-amino-1H-1,2,4-triazol-5-yl)amino]propoxy]phenylmethylamino]hexanamide
1-methyl-$N^5$-[3-[3-[(2-furanylmethyl)aminomethyl]phenoxy]propyl-1H-1,2,4-triazole-3,5-diamine
1-methyl-$N^5$-[3-[3-[(1H-pyrrol-2-yl-methyl)aminomethyl]phenoxy]propyl]-1H-1,2,4-triazole-3,5-diamine
5-[[2-[[[5-[[N-methyl-(2-furanylmethyl)amino]methyl]-2-furanyl]methyl]thio]ethyl]amino]-1-methyl-1H-1,2,4-triazole-3-methanol

and their physiologically acceptable salts.

According to one aspect ths invention provides compounds of formula (I) in which

$R_1$ represents a heteroaralkyl group made up of a heterocyclic part which is a furyl, thienyl, pyrrolyl, pyridinyl, pyrimidinyl, triazinyl, oxazolyl, triazolyl, thiazolyl, isoquinolinyl, quinolinyl or indolyl ring, which ring is unsubstituted or is substituted by a substituent selected from $C_{1-3}$ alkyl, $C_{1-3}$ alkoxy, hydroxy, hydroxy $C_{1-4}$ alkyl, amino $C_{1-4}$ alkyl, $C_{1-4}$ alkylamino $C_{1-4}$ alkyl, di-$C_{1-4}$ alkylamino $C_{1-4}$ alkyl and halogen, and which ring is linked to the alkyl part through carbon, and an alkyl part which is a straight or branched $C_{1-4}$ alkyl chain; and

$R_4$ represents $NR_7R_8$ where $R_7$ and $R_8$ each represent hydrogen, $C_{1-6}$ alkyl, $C_{1-6}$ alkyl substituted by hydroxy or $C_{1-3}$ alkoxy, $C_{3-6}$ alkenyl, phenyl $C_{1-3}$ alkyl or heteroaryl $C_{1-6}$ alkyl or $R_7$ and $R_8$ together represent $=CR_{12}R_{13}$ where $R_{12}$ represents phenyl or heteroaryl and $R_{13}$ represents hydrogen or $C_{1-6}$ alkyl (where heteroaryl within the definition of $R_4$ means furyl, pyridyl, thiazolyl or thienyl).

According to another aspect the invention provides compounds of formula (I) in which

$R_1$ is other than a heteroaralkyl group (as defined above); and

$R_4$ represents $NR_7R_8$ where $R_7$ and $R_8$ each represent hydrogen, $C_{1-6}$ alkyl, $C_{1-6}$ alkyl substituted by hydroxy or $C_{1-3}$ alkoxy, $C_{3-6}$ alkenyl, phenyl $C_{1-3}$ alkyl or heteroaryl $C_{1-6}$ alkyl or $R_7$ and $R_8$ together represent $=CR_{12}R_{13}$ where $R_{12}$ represents phenyl or heteroaryl and $R_{13}$ represents hydrogen or $C_{1-6}$ alkyl (where heteroaryl within the definition of $R_4$ means furyl, pyridyl, thiazolyl or thienyl).

It will be appreciated in the methods for the preparation of compounds of formula (I) given below, that for certain reaction steps it may be necessary to protect various reactive substituents in the starting materials for a particular reaction and subsequently to remove the protecting group. Such protection and subsequent deprotection may be particularly pertinent where $R_1$ and/or $R_2$ in intermediates used to prepare compounds of formula (I) are hydrogen atoms and/or when $R_3$ in intermediates is an alkyl group bearing a hydroxy substituent and/or when $R_4$ in certain intermediates is a free amino group. Standard protection and deprotection procedures can be employed:

for example formation of phthalimide (in the case of primary amines), benzyl, benzyloxycarbonyl, or trichloroethoxycarbonyl derivatives. Subsequent cleavage of the protection group is achieved by conventional procedures. Thus a phthalimide group may be cleaved by treatment with a hydrazine e.g. hydrazine hydrate or a primary amine for example methylamine; benzyl or benzyloxycarbonyl derivatives may be cleaved by hydrogenolysis in the presence of a catalyst, e.g. palladium, and trichloroethoxycarbonyl derivatives may be cleaved by treatment with zinc dust.

In describing the processes which may be used in preparing compounds of formula (I) or intermediates used in the preparation thereof, any of $R_1$ to $R_{13}$, Alk, Q, X, n and m are as defined in formula (I) unless otherwise stated.

Compounds of formula (I) where Alk represents $CH_2$ and in which $R_4$ is other than the group $—N=CR_{12}R_{13}$ may be prepared by reacting a compound of formula (III)

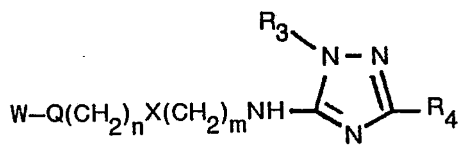

(III)

in which W represents the group —CHO with a reagent $R_1R_2NH$ followed by reduction of the intermediate thus formed with a hydride reducing agent such as an alkali or alkaline earth metal borohydride e.g. sodium borohydride, or aluminium hydride or lithium aluminium hydride or with hydrogen and a metal catalyst e.g. palladium or platinum.

The reaction may be effected by treatment of the compound of formula (III) with ammonia or an amine $R_1R_2NH$ in a solvent e.g. tetrahydrofuran or an alkanol such as ethanol or methanol, followed by the reduction.

Alternatively, for the production of compounds of formula (I) in which Alk is $C_{1-6}$ alkylene group and $R_4$ is other than the group $—N=CR_{12}R_{13}$, a compound of formula (IV)

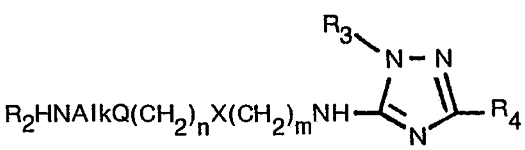

(IV)

may be reacted with an aldehyde $R_1^1CHO$ under the reducing conditions described above; the group $R_1^1$ having a meaning such that the resultant $R_1^1CH_2$— represents a group $R_1$ as defined above.

Compounds of formula (I) may also be prepared by reacting an intermediate of formula (IV) above with an appropriate halo compound such as a heteroaralkyl halide (e.g. 2-furylmethyl chloride).

Compounds of formula (I) in which $R_4$ represents the group $N=CR_{12}R_{13}$ may be prepared from compounds of formula (I) in which $R_4$ represents $NH_2$ by reaction with an aldehyde or ketone $R_{12}R_{13}CO$ in a solvent such as benzene, ethanol or methanol. The reaction is preferably carried out with heating, e.g. at reflux.

Compounds of formula (I) in which $R_4$ is an acyloxyalkyl group may be prepared by treating the corresponding hydroxyalkyl compound with an appropriate acid, e.g. acetic or benzoic acid at elevated temperatures e.g. 80—120°C in the absence or presence of a solvent such as toluene.

Compounds of formula (I) in which $R_4$ is the group $NR_7R_8$ where $R_8$ is $—COR_9$, $—SO_2R_{10}$ or $—C(=Y)NHR_{11}$ may be prepared by treating an amino triazole (V)

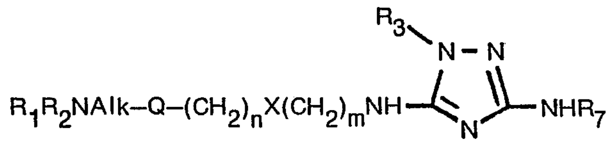

(V)

in which $R_1$, $R_2$, $R_3$ and $R_7$ are as defined in formula (I) or are groups readily convertible thereto with a reagent capable of replacing the hydrogen atom in the group $NHR_7$ by the group $R_8$ where $R_8$ is the group $COR_9$, $SO_2R_{10}$ or $C(=Y)NHR_{11}$.

Thus the aminotriazole (V) may be reacted with an activated derivative of either a carboxylic acid $R_9COOH$ or a sulphonic acid $R_{10}SO_3H$ to replace the hydrogen atom in the group $NHR_7$ by $—COR_9$ or $SO_2R_{10}$. Alternatively the aminotriazole (V) may be reacted with an isocyanate or isothiocyanate $R'_{11}NCY$ in which $R'_{11}$ has any of the meanings defined for $R_{11}$ in formula (I) except hydrogen or represents an alkali metal atom such as potassium or sodium or an alkoxycarbonyl group, e.g. ethoxycarbonyl, to replace the hydrogen atom in the group $NHR_7$ by $—C(=Y)NHR_{11}$.

Suitable activated derivatives include acid halides e.g. acid chlorides, alkylchloroformates, acid anhydrides including mixed anhydrides (e.g. acetic formic anhydride), or esters such as alkyl esters, ortho esters and (1-alkyl-2-pyridinyl) esters.

The reaction with an acid halide is preferably carried out in the presence of a base e.g. an inorganic base such as sodium hydroxide or an organic base such as triethylamine or pyridine. The reaction with an alkylchloroformate is preferably carried out in the presence of a base, e.g. potassium carbonate or triethylamine, in a solvent such as dimethylformamide. The reaction with an acid anhydride may be carried out in the absence or presence of a solvent such as pyridine.

In the reaction with an isocyanate or isothiocyanate, compounds of formula (I) in which $R_{11}$ is other than hydrogen are conveniently prepared by carrying out the reaction in a solvent such as acetonitrile at an elevated temperature, e.g. reflux. Compounds of formula (I) in which $R_{11}$ is hydrogen may be prepared by heating the aminotriazole (V) with an appropriate organicisocyanate or isothiocyanate such as ethylcarbonisothiocyanatidate, at an elevated temperature followed by hydrolysis of the resulting ester, for example with a base such as aqueous ethanolic sodium hydroxide.

Compounds of formula (I) in which $R_1$ is an alkylene chain interrupted by an amide grouping may be prepared by heating a compound of formula (I) in which $R_1$ is an alkylene chain substituted by an ester grouping $CO_2R_5$ (i.e. $R_1$ is the group $R_5OOC(CH_2)_x—$) with an amine $CH_3(CH_2)_yNH_2$ to give a compound of formula (I) in which $R_1$ is the group $CH_3(CH_2)_yNHCO(CH_2)_x—$ where x and y are integers and the total of x and y is not greater than 15.

Intermediates of formulae (III) and (IV) may in general be prepared by the methods referred to in British patent specification No. 2023133A and in European Patent specification publication No. 0016565.

Where the product of any of the above processes is a free base and a salt is required, the salt may be formed in a conventional manner. Thus for example, a generally convenient method of forming the salts is to mix appropriate quantities of the free base and the acid in an appropriate solvent(s) e.g. an alcohol such as ethanol or an ester such as ethyl acetate.

The invention is illustrated but not limited by the following Examples and Preparations.

In the Examples temperatures are in °C. Preparative and thin layer (t.l.c.) chromatography were carried out on silica using, unless otherwise stated, the following solvent system: ethyl acetate: water: isopropanol: 0.88 ammonia (25:8:15:2)

Preparation I

N-Butyl-6-aminohexanamide

N-Butyl-6-[[(phenylmethoxy)carbonyl]amino]hexanamide

A stirred solution of 6-[[(phenylmethoxy)carbonyl]amino]hexanoic acid (26.53 g) and dimethylformamide (10 drops) in dichloromethane (200 ml) was treated with thionylchloride (22 ml) at room temperature for 17 h. The solution was evaporated and the residue was azeotropically dried with toluene to give a yellow oil. Butylamine (50 ml) was added to a stirred solution of the yellow oil in dichloromethane (200 ml) at 0° over 0.5 h and the reaction was stirred at room temperature for 17 h. The solution was washed with water and evaporated to leave a solid which was washed with light petroleum b.p. 60—80° to give the *title compound* as a cream solid (30 g).

N.m.r. ($CDCl_3$) 2.70, s, (5H); 3.95, brt, (1H); 4.8, brt, (1H); 4.95, s, (2H); 6.8, m, (4H); 7.85, t, (2H); 8.1—8.9, m (10H); 9.10, t, (3H).

N-Butyl-6-aminohexanamide

Hydrogen, at atmospheric pressure, was passed through a stirred suspension of N-butyl-6-[[(phenylmethoxy)-carbonyl]amino]hexanamide (12.8 g) and palladium oxide an charcoal (1 g) in ethanol (150 ml) until uptake of hydrogen ceased. The suspension was filtered and evaporated to give an oil which was partitioned between ethyl acetate (100 ml) and 2N sodium hydroxide solution (100

ml). The organic extract was extracted with 2N hydrochloric acid which was basified to pH 12 with 2N sodium hydroxide solution and extracted with ethyl acetate. The organic extracts were evaporated to give the *title compound* as a cream semi solid (4 g) which was used without further purification.

Example 1

(a) 1-Methyl-$N^5$-[3-[3-[(3-pyridinylmethyl)amino methyl]phenoxy]propyl]-1H-1,2,4-triazole-3,5-diamine

A solution of $N^1$-cyano-N-[3-[3-(1,3-dioxolan-2-yl)phenoxy]propyl]-1-methyl-2-(phenylmethylene)hydrazinecarboximidamide (3.06 g) in tetrahydrofuran (60 ml) was stirred at room temperature for 0.5 h. with 2N hydrochloric acid (7 ml). The mixture was treated with 3-aminomethylpyridine (15 ml) and stirred at room temperature for a further 0.5 h. The mixture was treated with sodium borohydride (1.5 g), stirred at room temperature for 18 h. diluted with ethyl acetate, filtered and the filtrate evaporated *in vacuo*. The resulting oil was partitioned between ethyl acetate and water. The organic phase was evaporated *in vacuo* and the residue was purified by column chromatography on silica, using methanol. Evaporation of the eluates gave a brown oil which was triturated with diethyl ether to give the *title compound* as an off white solid (0.12 g) m.p. 114—7° T.l.c. $R_f$ 0.6

The following compounds were similarly prepared from $N^1$-cyano-N-[3-[3(1,3-dioxolan-2-yl)phenoxy]propyl]-1-methyl-2-(phenylmethylene)hydrazinecarboximidamide [A] and the corresponding amines.

(b) A (3.06 g) and furfurylamine (15 ml) gave 1-methyl-$N^5$-[3-[3-[(2-furanylmethyl)amino methyl]phenoxy]propyl]-1H-1,2,4-triazole-3,5-diamine as a white solid (0.77 g). m.p. 91.5—93°.

| | | | |
|---|---|---|---|
| Assay found: | C, 60.45: | H, 6.76: | N, 23.06; |
| $C_{18}H_{24}N_6O_2$ requires: | C, 60.66; | H. 6.79; | N, 23.58% |

(c) A (3.06 g) and methyl, 11-aminoundecanoate (4.85 g) gave methyl, 11-[3-[3-[(1-methyl-3-amino-1H-1,2,4-triazol-5-yl)amino]propoxy]phenyl methyl amino] undecanoate as a white solid (0.89 g) m.p. 83.5—85°.

| | | | |
|---|---|---|---|
| Assay found: | C, 63.20; | H, 8.86; | N, 17.56; |
| $C_{25}H_{42}N_6O_3$ requires: | C, 63.26; | H, 8.92; | N, 17.71% |

(d) A (2.2 g) and dodecylamine (10 ml) gave 1-methyl-$N^5$-[3-[3-[(dodecylamino)methyl]phenoxy]-propyl]-1H-1,2 4-triazole-3,5-diamine as a white solid (0.5 g) m.p. 85—86°.

| | | | |
|---|---|---|---|
| Assay found: | C, 67.05; | H, 9.73; | N, 18.46; |
| $C_{25}H_{44}N_6O$ requires: | C, 67.56; | H, 9.90; | N, 18.91% |

(e) A (2.03 g) and hexadecylamine (11 g) gave 1-methyl-$N^5$-[3-[3-[(hexadecyl-amino)methyl]phenoxy]propyl]-1H-1,2,4-triazole-3,5-diamine as a white solid (0.1 g) m.p. 88—90.5°, after recrystallisation from a mixture of ethyl acetate and light petroleum b.p. 60—80° (1:4).

| | | | |
|---|---|---|---|
| Assay found: | C, 68.62; | H, 10.11; | N, 16.12; |
| $C_{29}H_{52}N_6O . 0.5H_2O$ requires: | C, 68.33; | H, 10.48; | N, 16.49% |

(f) A (3.06 g) and methyl-(2-amino)acetate (3 g) gave methyl-[3-[3-[(1-methyl-3-amino-1H-1,2,4-triazol-5-yl)-amino]propoxy]phenylmethylamino]acetate as an oil (590 mg) purified by chromatography on silica using methanol/ethyl acetate (1:1).

T.l.c. silica ethyl acetate: methanol (1:1) $R_f$ 0.5

N.m.r. ($CDCl_3$) 2.75 t, (1H); 3.0—3.3, m, (3H); 5.66, t, (1H); 5.90, t, (2H); 6.10 br.s. (2H); 6.25, s, (2H); 6.30 s (3H); 6.48, q. (2H); 6.60 s (2H); 6.65, s, (3H); 7.78, br. s, (1H); 7.88, m, (2H).

(g) A (3.06 g) and N-butyl-(6-amino)hexanamide (3.7 g) gave N-butyl-[3-[3-[(1-methyl-3-amino-1H-1,2,4-triazol-5-yl)amino]propoxy]phenylmethylamino]hexanamide dihydrate as a brown oil (700 mg).

N.m.r. ($CDCl_3$) 2.8, t, (1H); 3.1—3.4, m, (3H); 4.4, m, (1H); 5.6, m, (1H); 5.98, t, (2H); 6.2, br, (2H); 6.32, s, (2H); 6.50, q, (2H); 6.68, s, (3H); 6.83, q, (2H); 7.45, t, (2H); 7.8—8.1, m, (4H); 8.2—9.0, m, (11H); 9.15, t, (3H)

| | | | |
|---|---|---|---|
| Assay found: | C, 57.66; | H, 8.20; | N, 19.82; |
| $C_{23}H_{29}N_7O_2 . 2H_2O$ requires: | C, 57.48; | H, 8.80; | N, 20.39% |

Example 2

(a) 5-[[3-[3-[(3-Amino-1-methyl-1,2,4-triazol-5-yl)-amino]propoxy]phenylmethyl]aminomethyl]furan-2-methanol

A solution of 1-methyl-$N^5$-[3-[3-(aminomethyl)phenoxy]propyl]-1H-1,2,4-triazole-3,5-diamine (830 mg) and 2-hydroxymethylfuran-5-carboxaldehyde (380 mg) in ethanol (30 ml) was stirred at room temperature for 6 h and treated with sodium borohydride (1 g) for 16.5 h. The mixture was quenched with water, partially evaporated and extracted with ethyl acetate. The organic extracts were evaporated to give a yellow oil which was purified by preparative layer chromatography to give the *title compound* as a white solid (400 mg) m.p. 120—122°.

N.m.r. ($CDCl_3$/DMSO) 2.79, t, (1H); 3—3.3, m, (3H); 3.87, dd, (2H); 4.13, t, (1H); 5.5 s (2H); 5.70, br. s. (2H); 5.95, t, (2H); 6.27, s, (2H); 6.29, s, (2H); 6.4—6.7 m (2H); 6.68 s (3H); 7.75—8.10, m, (3H).

The following compounds were similarly prepared from 1-methyl-$N^5$-[3-[3-(amino methyl)phenoxy]propyl]-1H-1,2,4-triazole-3,5-diamine [A] and the corresponding heterocyclic aldehydes.

(b) A (276 mg) and thiophene-2-carboxaldehyde (0.1 ml) gave 1-methyl-$N^5$-[3-[3-[(2-thienylmethyl)aminomethyl]phenoxy]propyl]-1H-1,2,4-triazole-3,5-diamine as a white solid (24 mg) m.p. 96.5—8°.

| | | | |
|---|---|---|---|
| Assay found: | C, 57.60; | H, 6.45; | N, 22.27; |
| $C_{18}H_{24}N_6OS$ requires: | C, 58.04; | H, 6.49; | N, 22.56% |

(c) A (830 mg) and 2-methylfuran-5-carboxaldehyde (0.3 ml) gave 1-methyl-$N^5$-[3-[3-[(2-methyl-5-furanyl-methyl)aminomethyl]phenoxy]propyl]-1H-1,2,4-triazole-3,5-diamine which was isolated as its tartrate salt (1.15 g) m.p. softens ca. 95°.

N.m.r. ($D_2O$) 2.53 t, (1H); 2.8—3.0, m, (3H); 3.46, d, (1H); 3.87, d, (1H); 5.53. s (2H); 5.76 s (2H); 5.8. s. (2H); 5.82. t. (2H); 6.5. t, (2H); 6.6, s. (3H); 7.68. s (3H); 7.85 m (2H).

(d) A (830 mg) and pyrrole-2-carboxaldehyde (286 mg) gave 1-methyl-$N^5$-[3-[3-[(1H-pyrrol-2-yl-methyl)amino-methyl]phenoxy]propyl]-1H-1,2,4-triazole-3,5-diamine which was isolated as its hydrated tartrate salt (420 mg).

N.m.r. ($D_2O$) 2.55, t, (1H); 2.8—3.05, m, (4H); 3.60, m, (2H); 3.72, t, (1H); 5.60, s, (2H); 5.73, s, (2H); 5.83, s+t, (4H); 6.55, t, (2H); 6.65 s, (3H); 7.93 m (2H).

| | | | |
|---|---|---|---|
| Assay found | C, 50.99; | H, 6.28; | N, 18.74; |
| $C_{22}H_{31}N_7O_7 . H_2O$ requires: | C, 50.47; | H, 6.35; | N, 18.72% |

Example 3

N-Decyl-[3-[3-[(1-methyl-3-amino-1H-1,2,4-triazol-5-yl)amino]propoxy]phenylmethylamino]acetamide

A mixture of methyl-[3-[3-[(1-methyl-3-amino-1H-1,2,4-triazol-5-yl)amino]propoxy]phenylmethylamino]acetate (110 mg) and decylamine (0.5 ml) was heated at 100° for 16 h. The crude reaction mixture was purified by preparative layer chromatography to give the *title compound* as a yellow oil (120 mg).

T.l.c. $R_f$ 0.8.

N.m.r. ($D_2O$) 2.6—3.3, m, (5H); 5.72, br.t, (1H); 5.93, t, (2H); 6.18, br.s, (2H); 6.30, s, (2H); 6.47, q, (2H); 6.65, s, (3H); 6.72, s+m, (4H); 7.88, m, (2H); 8.0—8.9, m, (17H); 9.15, br.t, (3H).

Example 4

(a) 1-Methyl-5-[[3-[3-[[(2-furanylmethyl)amino]methyl]phenoxy]propyl]amino]-1H-1,2,4-triazole-3-methanol

A solution of 3-[3-[(3-hydroxymethyl-1-methyl-1,2,4-triazol-5-yl)amino]propoxy]benzaldehyde (871 mg) and furfurylamine (5.3 ml) in ethanol (50 ml) was stirred at room temperature for 6 h, then treated with sodium borohydride (1 g) for 17 h. The mixture was quenched with water, partially evaporated and extracted with ethyl acetate. The organic extract was evaporated and purified by column chromatography on silica using methanol/ethyl acetate (1:1) as eluant to give the *title compound* as a red oil (500 mg).

T.l.c system A Rf 0.7. The tartrate salt was formed in ethyl acetate m.p. softens at 70°

N.m.r. ($D_2O$) 2.35, d, (1H); 2.55 dt, (1H); 2.83—3.1, m, (3H); 3.38, d, (1H); 3.5, dd, (1H); 5.56, s, (2H), 5.6, s, (2H); 5.7, s, (2H); 5.8, t, (2H); 5.82, s, (2H); 6.5 s+t; (5H); 7.9, m, (2H);

(b) Similarly, 3-[3-[(3-hydroxymethyl-1-methyl-1,2,4-triazole-5-yl)amino]propoxy]benzaldehyde (790 mg) and dodecylamine (8 ml) gave 1-methyl-5-[[3-[3-[(dodecyl)aminomethyl]phenoxy]propyl]amino]-1H-1,2,4-triazole-3-methanol as a cream solid (550 mg) m.p. 55—57°.

N.m.r. ($CDCl_3$) 2.76, t, (1H); 3.0—3.35, m, (3H); 5.48, s, (2H); 5.88, t, (2H); 6.23, s, (2H); 6.40, q, (2H); 6.50, s, (3H); 7.38, t, (2H); 7.60, brs. (2H); 7.88, q, (2H); 8.3—8.9 m, (20H); 9.13, m, (3H).

Example 5
1-Methyl-$N^3$-(4-pyridylmethylene)-$N^5$-[3-[3-[(2-furanylmethyl)-aminomethyl)phenoxy]propyl]-1H-1,2,4-triazole-3,5-diamine

A solution of 1-methyl-$N^5$-[3-[3-[(2-furanylmethyl)aminomethyl]phenoxy]propyl]-1H-1,2,4-triazole-3,5-diamine (310 mg) and 4-pyridinecarboxaldehyde (0.17 ml) in toluene (15 ml) was heated at reflux for 30 h. The solution was cooled to give the *title compound* as a yellow solid (250 mg) m.p. 90—94°.

N.m.r. ($CDCl_3$) 0.92, s, (1H); 1.25, m, (2H); 2.20, m, (2H); 2.6—2.9, t+m, (2H); 3.0—3.3, m, (3H); 3.7, m, (1H); 3.85, m, (1H); 5.4 t, (1H); 5.84 t, (2H); 6.1—6.5, m, (9H); 7.8. m, (2H); 8.2, s, (1H).

Example 6
5-[[2-[[[5-[[N-Methyl(2-furanylmethyl)amino]methyl]-2-furanyl]methyl]thio]ethyl]amino]-1-methyl-1H-1,2,4-triazole-3-methanol

5-[[2-[[[5-(methylamino)methyl-2-furanyl]methyl]thio]ethyl]amino]-1-methyl-1H-1,2,4-triazole-3-methanol

A solution of 2-[[[5-(methylamino)methyl-2-furanyl]methyl]thio]ethanamine (4.0 g) and methyl N-[2-(acetoxy)acetyl]-1-methyl]-2-(phenylmethylene)hydrazine carboximidothioate (6.2 g) in acetonitrile (25 ml) was stirred for 3 h at 25°C.

The solution was evaporated and the residue was chromatographed on silica with ethyl acetate/methanol (4:1) to give the *title compound* as a gum (3.5 g). T.l.c. silica methanol. Rf 0.45. A portion of this material was converted to its oxalate salt m.p. 157—9°.

5-[[2-[[[5-[[N-Methyl-(2-furanylmethyl)amino]methyl]-2-furanyl]methyl]thio]ethyl]amino]1-methyl-1H-1,2,4-triazole-3-methanol

A solution of 5-[[2-[[[5-(methylamino)methyl-2-furanyl]methyl]thio]ethyl]amino]-1-methyl-1H-1,2,4-triazole-3-methanol (0.5 g) and 2-furaldehyde (0.31 g) in ethanol (20 ml) was stirred at 25°C for 2 h then treated with sodium borohydride (38 mg). After 2 h 2-furaldehyde (0.31 g) was added and after a further 2h sodium borohydride (76 mg) was added. The resulting solution was stirred for 18 h, treated with acetic acid (2 ml) and evaporated *in vacuo*. The residue was dissolved in water which was basified with potassium carbonate and extracted with ethyl acetate. The organic extracts were evaporated to give a gum which was chromatographed on silica with ethyl acetate:methanol (95:5) to give the *title compound* as a gum (0.29 g).

T.l.c. silica; ethyl acetate: ethanol: 0.88 ammonia (20:3:2); Rf 0.37.

N.m.r. ($CDCl_3$) 2.60, m, (1H); 3.70 s+m, (4H); 5.48, s+t, (3H); 6.3—6.6,4 s+m, (12H); 7.20, t, (2H); 7.75, s, (3H).

Examples of pharmaceutical compositions

| Tablets | mg/tablet |
|---|---|
| Active ingredient | 100.00 |
| Microcrystalline Cellulose BPC | 198.50 |
| Magnesium stearate BP | 1.50 |
| Compression weight | 300.00 |

The active ingredient is sieved through a 250 $\mu$m sieve, blended with the excipients and compressed using 9.5 mm punches. Tablets of other strengths may be prepared by altering the compression weight and using punches to suit.

The tablets may be film coated with suitable film forming materials, e.g. methyl cellulose or

hydroxypropyl methyl cellulose using standard techniques. Alternatively the tablets may be sugar coated.

Injection for intravenous administration

| | % w/v |
|---|---|
| Active ingredient | 1.00 |
| Water for injections B.P. to | 100.00 |

Sodium chloride may be added to adjust the tonicity of the solution and the pH may be adjusted to that of maximum stability and/or to facilitate solution of the active ingredient using either dilute acid of alkali.

The solution is prepared, clarified and filled into appropriate sized ampoules sealed by fusion of the glass. The injection is sterilised by heating in an autoclave using one of the acceptable cycles. Alternatively the solution may be sterilised by filtration and filled into sterile ampoules under aseptic conditions. The solution may be packed under an inert atmosphere of nitrogen.

The ability of the compounds of the invention to inhibit histamine induced gastric acid secretion has been demonstrated in the perfused rat stomach preparation referred to previously; the effectiveness of a compound conveniently being expressed in terms of its $ED_{50}$ (mg/kg) value. The results obtained for a representative number of compounds according to the invention are given below:

| | |
|---|---|
| 1 (b) | 0.095 |
| 1 (d) | 0.075 |
| 1 (g) | 0.25 |
| 2 (a) | 0.19 |
| 2 (d) | 0.014 |
| 5 | 0.18 |
| 6 | 0.077 |

The compounds of the invention have in general low toxicity at doses at which they effectively inhibit gastric acid secretion. Thus for instance the compounds listed above produced no toxic effects when administered intravenously to anaesthetised rats at doses of at least 4 times their $ED_{50}$ values.

## Claims

1. Compounds of the general formula (I)

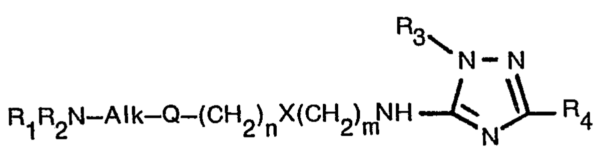

(I)

and physiologically acceptable salts and hydrates thereof, in which

$R_1$ represents a heteroaralkyl group made up of a heterocyclic part which is a furyl, thienyl, pyrrolyl, pyridinyl, pyrimidinyl, triazinyl, oxazolyl, triazolyl, thiazolyl, isoquinolinyl, quinolinyl or indolyl ring, which ring is unsubstituted or is substituted by a substituent selected from $C_{1-3}$ alkyl, $C_{1-3}$ alkoxy, hydroxy, hydroxy $C_{1-4}$ alkyl, amino $C_{1-4}$ alkyl, $C_{1-4}$ alkylamino $C_{1-4}$ alkyl, di-$C_{1-4}$ alkylamino $C_{1-4}$ alkyl and halogen, and which ring is linked to the alkyl part through carbon, and an alkyl part which is a straight or branched $C_{1-4}$ alkyl chain, or

$R_1$ represents a $C_{11-16}$ straight or branched saturated or unsaturated alkylene chain optionally substituted by a hydroxy group or $R_1$ represents a $C_{1-16}$ straight or branched saturated or unsaturated alkylene chain substituted by an ester group $CO_2R_5$ or an amide group $CONH_2$, wherein $R_5$ is a $C_{1-3}$ alkyl group, or $R_1$ represents a $C_1$—$C_{16}$ straight or branched saturated or unsaturated alkylene chain interrupted by an oxygen atom, a sulphur atom, a sulphoxide group, a sulphone group, an amide group (—CONH— or —NHCO—) or an ester group (—CO—O— or O—CO—) with the provisos that $R_1$ does not represent a $C_{1-6}$ alkyl group substituted by a $C_{1-6}$ alkoxy group and that when the alkylene chain is interrupted by an oxygen or sulphur atom or a sulphoxide, amide (CONH) or ester

(COO) group there must be at least two carbon atoms between that group and the nitrogen atom to which $R_1$ is attached;

$R_2$ represents hydrogen or a $C_{1-4}$ alkyl group;

Alk represents a straight or branched alkylene chain of 1 to 6 carbon atoms;

Q represents a furan or thiophen ring in which incorporation into the rest of the molecule is through bonds at the 2- and 5-positions, the furan ring optionally bearing a further substituent $R_6$ adjacent to the group $R_1R_2$NAlk-, or Q represents a benzene ring in which incorporation into the rest of the molecule is through bonds at the 1- and 3- or 1- and 4-positions;

$R_6$ represents halogen or $C_{1-4}$ alkyl which may be substituted by hydroxy or $C_{1-4}$ alkoxy;

X represents —$CH_2$—, —O—, —S—, or —NH—;

n represents zero, 1 or 2;

m represents 2, 3 or 4;

$R_3$ represents hydrogen, $C_{1-6}$ alkyl, $C_{3-6}$ alkenyl or $C_{2-6}$ alkyl substituted by hydroxy or $C_{1-6}$ alkoxy; and

$R_4$ represents hydrogen, $C_{1-6}$ alkyl, $C_{3-6}$ alkenyl, phenyl $C_{1-3}$ alkyl, hydroxy $C_{1-6}$ alkyl, $C_{1-6}$ alkanoyloxy $C_{1-6}$ alkyl, $C_{1-6}$ alkoxy $C_{1-6}$ alkyl, amino $C_{1-6}$ alkyl, $C_{1-6}$ alkylamino $C_{1-6}$ alkyl, di $C_{1-6}$ alkylamino $C_{1-6}$ alkyl, hydroxy or $C_{1-6}$ alkoxy or the group $NR_7R_8$ where

$R_7$ represents hydrogen, $C_{1-6}$ alkyl, $C_{1-6}$ alkyl substituted by hydroxy or $C_{1-3}$ alkoxy, $C_{3-6}$ alkenyl, phenyl $C_{1-3}$ alkyl or heteroaryl $C_{1-6}$ alkyl and $R_8$ represents any of the groups defined for $R_7$ or may represent the group $COR_9$ where $R_9$ represents hydrogen, $C_{1-6}$ alkyl, phenyl optionally substituted by a $C_{1-3}$ alkyl or $C_{1-3}$ alkoxy group, $C_{1-6}$ alkoxy, heteroaryl or heteroaryl $C_{1-6}$ alkyl or $R_8$ represents the group $SO_2R_{10}$ where $R_{10}$ represents $C_{1-6}$ alkyl or phenyl optionally substituted by a $C_{1-3}$ alkyl or $C_{1-3}$ alkoxy group, or $R_8$ represents the group

$$\underset{\underset{\displaystyle Y}{\|}}{C}-NHR_{11}$$

where Y is oxygen or sulphur and

$R_{11}$ represents hydrogen, $C_{1-6}$ alkyl, $C_{3-8}$ cycloalkyl, or phenyl optionally substituted by a $C_{1-3}$ alkyl or $C_{1-3}$ alkoxy group, or

$R_7$ and $R_8$ taken together represent the group $=CR_{12}R_{13}$ where $R_{12}$ represents phenyl or heteroaryl and $R_{13}$ represents hydrogen or $C_{1-6}$ alkyl with the provisos that

(1) when X is —NH— then n is zero, and

(2) in the definition of the group $R_4$ the term "heteroaryl" means furyl, pyridyl, thiazolyl or thienyl.

2. Compounds as claimed in Claim 1 in which the groups $R_1$, $R_2$ $R_3$ and $R_4$ have the following meanings:

$R_1$: a heteroaralkyl group in which the heterocyclic portion is a furyl, thienyl, pyrrolyl, pyridinyl, pyrimidinyl, triazinyl, oxazolyl, triazolyl, thiazolyl, isoquinolinyl, quinolinyl or indolyl ring, which ring is unsubstituted or substituted by a substituent selected from $C_{1-3}$ alkyl, $C_{1-3}$ alkoxy, hydroxy, hydroxy $C_{1-4}$ alkyl, amino $C_{1-4}$ alkyl, $C_{1-4}$ alkylamino $C_{1-4}$ alkyl, di-$C_{1-4}$ alkylamino $C_{1-4}$ alkyl and halogen, and the alkylene portion is a methylene, ethylene or propylene grouping; or an alkylene chain which is saturated and contains up to 16 carbon atoms or is substituted by or interrupted by an amide or ester grouping;

$R_2$: hydrogen or a methyl or ethyl group;

$R_3$: hydrogen $C_{1-4}$ alkyl, or hydroxy $C_{2-4}$ alkyl;

$R_4$: hydrogen, hydroxy, $C_{1-4}$ alkyl, hydroxy $C_{1-4}$ alkyl, $C_{1-3}$ alkoxy $C_{1-4}$ alkyl, phenyl $C_{1-3}$ alkyl, $C_{2-4}$ alkanoyloxy $C_{1-4}$ alkyl, amino $C_{1-4}$ alkyl, amino, $C_{1-4}$ alkylamino or di-$C_{1-4}$ alkylamino, phenyl $C_{1-3}$ alkylamino, or a heteroaryl $C_{1-3}$ alkylamino group where the heteroaryl ring is furyl, pyridyl, thiazolyl or thienyl; or the group $NHCOR_9$ where $R_9$ represents hydrogen $C_{1-3}$ alkyl, $C_{1-3}$ alkoxy, furyl, pyridyl, thiazolyl, thienyl, or phenyl optionally substituted by a $C_{1-3}$ alkyl or $C_{1-3}$ alkoxy group; or the group $NHSO_2R_{10}$ where $R_{10}$ represents $C_{1-3}$ alkyl or phenyl optionally substituted by a $C_{1-3}$ alkyl or $C_{1-3}$ alkoxy group; or the group $NHCONHR_{11}$ where $R_{11}$ is $C_{1-3}$ alkyl, $C_{5-7}$ cycloalkyl or phenyl optionally substituted by a $C_{1-3}$ alkyl or $C_{1-3}$ alkoxy group; or the group $N{=}CHR_{12}$ where $R_{12}$ is a phenyl or pyridyl group;

the group Alk is $(CH_2)_p$ where p is 1, 2 or 3; and the group Q is a benzene ring incorporated into the rest of the molecule through bonds at the 1- and 3- or 1- and 4-positions, with n as zero, X as oxygen and m as 3 or 4; or the group Q is a furan or thiophene ring with n as 1, X as sulphur and m as 2, with the furan ring optionally containing a further substituent $R_6$ where $R_6$ is bromine, $C_{1-3}$ alkyl or $C_{1-3}$ alkoxy-$C_{1-3}$ alkyl; or the group Q is a furan ring with n as 1, X as $CH_2$ and m as 2.

3. Compounds as claimed in Claim 2 in which the groups $R_1$, $R_2$, $R_3$ and $R_4$ have the following meanings:

$R_1$: a heteroarylmethyl group where the heteroaryl ring is furyl, thienyl, pyrrolyl or pyridinyl linked to the methyl portion via a carbon atom; or a $C_{11-16}$ straight or branched saturated alkylene chain; or a $C_{1-16}$ straight or branched saturated alkylene chain interrupted by an amide (—NHCO—) group;
$R_2$: hydrogen or a methyl or ethyl group;
$R_3$: hydrogen, methyl, ethyl or 2-hydroxyethyl;
$R_4$: hydroxy, phenyl $C_{1-3}$ alkyl, $C_{1-4}$ alkyl substituted by hydroxy, $C_{1-3}$ alkoxy, $C_{2-4}$ alkanoyloxy or amino; amino, di-$C_{1-4}$ alkylamino, $NHCOR_9$ where $R_9$ represents hydrogen, $C_{1-3}$ alkoxy or phenyl, $NHCONHR_{11}$ where $R_{11}$ represents phenyl, or $N{=}CHR_{12}$ where $R_{12}$ is phenyl or pyridyl;
the group Alk is a methylene or ethylene group; and the group Q is a benzene ring incorporated into the rest of the molecule through bonds at the 1- and 3- or 1- and 4-positions, with n as zero, X as oxygen and m as 3 or 4; or the group Q is a furan or thiophene ring with n as 1, X as sulphur and m as 2, with the furan ring optionally containing a further substituent $R_6$ where $R_6$ is bromine, $C_{1-3}$ alkyl or $C_{1-3}$ alkoxy-$C_{1-3}$ alkyl; or the group Q is a furan ring with n as 1, X as $CH_2$ and m as 2.

4. Compounds as claimed in any of claims 1 to 3 in which the group Q is a benzene ring incorporated into the rest of the molecule through bonds at the 1- and 3-positions, with n as zero, X as oxygen and m as 3 or 4; or the group Q is a furan ring optionally containing a further substituent $R_6$ where $R_6$ is $C_{1-4}$ alkyl, with n as 1, X as sulphur and m as 2.

5. Compounds as claimed in claims 1 or 2 in which $R_1$ is a heteroaralkyl group, made up of a heterocyclic part which is a furyl, thienyl, pyrrolyl, pyridinyl, pyrimidinyl, triazinyl, oxazolyl, triazolyl, thiazolyl, isoquinolinyl, quinolinyl as indolyl ring, which ring is unsubstituted or is substituted by a substituent selected from $C_{1-3}$ alkyl, $C_{1-3}$ alkoxy, hydroxy, hydroxy $C_{1-4}$ alkyl, amino $C_{1-4}$ alkyl, $C_{1-4}$ alkylamino $C_{1-4}$ alkyl, di-$C_{1-4}$ alkylamino $C_{1-4}$ alkyl and halogen, and which ring is linked to the alkyl part through carbon, and an alkyl part which is a straight or branched $C_{1-4}$ alkyl chain.

6. Compounds as claimed in Claim 5, corresponding to formula (II)

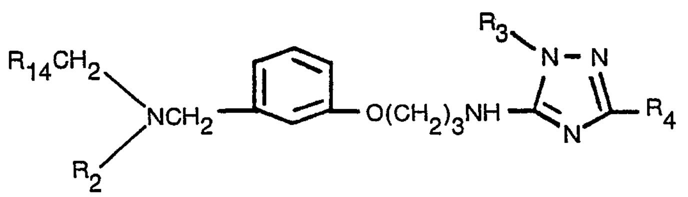

(II)

where $R_{14}$ is a furyl, thienyl, pyrrolyl or pyridinyl ring linked through a carbon atom, $R_2$ is hydrogen or methyl; $R_3$ is hydrogen or methyl, and $R_4$ is an amino, hydroxy $C_{1-6}$ alkyl, $C_{1-6}$ alkanoyloxy $C_{1-6}$ alkyl, $C_{1-6}$ alkoxy $C_{1-6}$ alkyl, amino $C_{1-6}$ alkyl, benzyl, formamido, $C_{1-6}$ alkoxycarbonylamino, $C_{2-7}$ alkanoylamino, hydroxy, aroylamino (where the aryl group is phenyl optionally substituted by a $C_{1-3}$ alkyl or $C_{1-3}$ alkoxy group), phenylcarbamoylamino or pyridylmethylenamino group.

7. Compounds as claimed in claim 6, in which $R_4$ represents amino, hydroxy $C_{1-6}$ alkyl or pyridylmethylenamino.

8. 5-[[2-[[[5-[[N-Methyl-(2-furanylmethyl)amino]methyl]-2-furanyl]methyl]thio]ethyl]amino]-1-methyl-1H-1,2,4-triazole-3-methanol and its physiologically acceptable salts.

9. 1-Methyl-$N^5$-[3-[3-[(dodecylamino)methyl]phenoxy]propyl]-1H-1,2,4-triazole-3,5-diamine and its physiologically acceptable salts.

10. 1-Methyl-$N^5$-[3-[3-[(2-furanylmethyl)aminomethyl]phenoxy]propyl]-1H-1,2,4-triazole-3,5-diamine, 1-methyl-$N^5$-[3-[3-[(1H-pyrrol-2-yl-methyl)aminomethyl]phenoxy]propyl]-1H-1,2,4-triazole-3,5-diamine and their physiologically acceptable salts.

11. Compounds as claimed in Claim 1 in which

$R_1$ represents a heteroaralkyl group made up of a heterocyclic part which is a furyl, thienyl, pyrrolyl, pyridinyl, pyrimidinyl, triazinyl, oxazolyl, triazolyl, thiazolyl, isoquinolinyl, quinoylinyl or indolyl ring, which ring is unsubstituted or is substituted by a substituent selected from $C_{1-3}$ alkyl, $C_{1-3}$ alkoxy, hydroxy, hydroxy $C_{1-4}$ alkyl, amino $C_{1-4}$ alkyl, $C_{1-4}$ alkylamino $C_{1-4}$ alkyl, di-$C_{1-4}$ alkylamino $C_{1-4}$ alkyl and halogen, and which ring is linked to the alkyl part through carbon, and an alkyl part which is a straight or branched $C_{1-4}$ alkyl chain; and

$R_4$ represents $NR_7R_8$ where $R_7$ and $R_8$ each represent hydrogen, $C_{1-6}$ alkyl, $C_{1-6}$ alkyl substituted by hydroxy or $C_{1-3}$ alkoxy, $C_{3-6}$ alkenyl, phenyl $C_{1-3}$ alkyl or heteroaryl $C_{1-6}$ alkyl or $R_7$ and $R_8$ together represent $=CR_{12}R_{13}$ where $R_{12}$ represents phenyl or heteroaryl and $R_{13}$ represents hydrogen or $C_{1-6}$ alkyl (where heteroaryl within the definition of $R_4$ means furyl, pyridyl, thiazolyl or thienyl).

12. A process for the production of compounds of formula (I) as defined in claim 1 which comprises:

(a) for the production of compounds in which Alk is $CH_2$ and $R_4$ is other than $—N{=}CR_{12}R_{13}$, reacting a compound of formula (III)

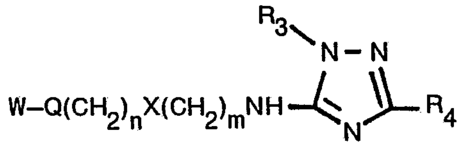

(III)

in which W represents the group —CHO, with a reagent of formula $R_1R_2NH$, followed by reduction of the intermediate thus formed with a hydride reducing agent or hydrogen and a metal catalyst;

(b) for the production of compounds in which $R_4$ is other than $—N{=}CR_{12}R_{13}$, reacting a compound of formula (IV)

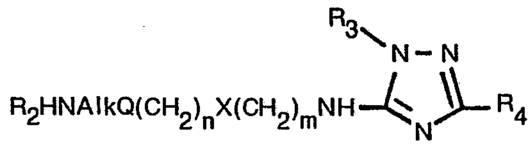

(IV)

with an aldehyde $R_1^1CHO$ under reducing conditions provided by a hydride reducing agent or hydrogen and a metal catalyst, where the group $R_1^1$ has a meaning such that the resulting group $R_1^1CH_2$— represents a group $R_1$;

(c) reacting a compound of formula (IV) as defined above with a compound $R_1Hal$ where Hal is halogen;

(d) for the production of compounds in which $R_4$ represents $N{=}CR_{12}R_{13}$, reacting a compound of formula (I) in which $R_4$ is $NH_2$ with an aldehyde or ketone $R_{12}R_{13}CO$;

(e) for the production of compounds in which $R_4$ represents an acyloxyalkyl group, reacting a compound in which $R_4$ represents hydroxyalkyl with an appropriate acid;

(f) for the production of compounds in which $R_4$ is the group $NR_7R_8$ where $R_8$ is $—COR_9$, $—SO_2R_{10}$ or $—C({=}Y)NHR_{11}$, treating an aminotriazole (V)

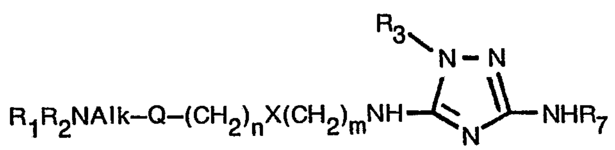

(V)

in which $R_1$, $R_2$, $R_3$ and $R_7$ are as defined in formula (I) or are groups readily convertible thereto, with

(i) an activated derivative of either a carboxylic acid $R_9COOH$ or a sulphonic acid $R_{10}SO_3H$ to replace the hydrogen atom in the group $NHR_7$ by $—COR_9$ or $—SO_2R_{10}$; or

(ii) an isocyanate or isothiocyanate $R'_{11}NCY$ in which $R'_{11}$ has any of the meanings defined for $R_{11}$ in formula (I) except hydrogen or represents an alkali metal atom or an alkoxycarbonyl group, to replace the hydrogen atom in the group $NHR_7$ by $—C({=}Y)NHR_{11}$;

(g) for the production of compounds in which $R_1$ is an alkylene chain interrupted by an amide grouping, treating a compound of formula (I) in which $R_1$ is an alkylene chain substituted by an ester grouping $CO_2R_5$ (i.e. $R_1$ is the group $R_5OOC\,(CH_2)_x$—) with an amine $CH_3(CH_2)_yNH_2$ to give a compound of formula (I) in which $R_1$ is the group $CH_3(CH_2)_yNHCO(CH_2)_x$— where x and y are integers and the total of x and y is not greater than 15;

and where the compound of formula (I) is produced in the form of a free base, optionally converting the free base into a salt.

13. A pharmaceutical composition comprising a compound as claimed in claims 1 to 11 together with at least one pharmaceutically acceptable carrier or diluent, and optionally one or more further active ingredients.

**Patentansprüche**

1. Verbindungen der allgemeinen Formel (I):

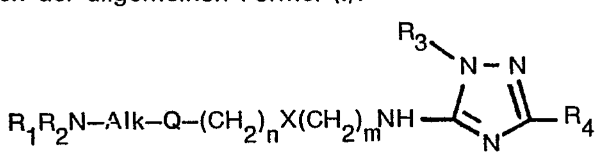

(I)

und die physiologisch annehmbaren Salze und Hydrate davon, worin $R_1$ für eine Heteroaralkylgruppe steht, die aus einem heterocyclischen Teil, nämlich einem Furyl-, Thienyl-, Pyrrolyl-, Pyridinyl-, Pyrimidinyl-, Triazinyl-, Oxazolyl-, Triazolyl-, Thiazolyl-, Isochinolinyl-, Chinolinyl- oder Indolylring, welcher Ring unsubstituiert ist oder durch einen Substituenten, ausgewählt aus $C_{1-3}$-Alkyl, $C_{1-3}$-Alkoxy, Hydroxy, Hydroxy-$C_{1-4}$-alkyl, Amino-$C_{1-4}$-alkyl, $C_{1-4}$-Alkylamino-$C_{1-4}$-alkyl, Di-$C_{1-4}$-alkylamino-$C_{1-4}$-alkyl und Halogen, substituiert ist, und wobei dieser Ring mit dem Alkylteil durch Kohlenstoff verbunden ist, und einem Alkylteil, nämlich einer geraden oder verzweigten $C_{1-4}$-Alkylkette, besteht,

oder $R_1$ für eine gerade oder verzweigte, gesättigte oder ungesättigte $C_{11-16}$-Alkylenkette, die gegebenenfalls durch eine Hydroxygruppe substituiert ist, steht oder $R_1$ für eine gerade oder verzweigte, gesättigte oder ungesättigte $C_{1-16}$-Alkylenkette, die durch eine Estergruppe $CO_2R_5$ oder eine Amidgruppe $CONH_2$ substituiert ist, wobei $R_5$ eine $C_{1-3}$-Alkylgruppe ist, steht oder $R_1$ eine gerade oder verzweigte, gesättigte oder ungesättigte $C_1$—$C_{16}$-Alkylenkette, die durch ein Sauerstoffatom, ein Schwefelatom, eine Sulfoxidgruppe, eine Sulfongruppe, eine Amidgruppe (—CONH— oder —NHCO—) oder eine Estergruppe (—CO—O— oder —O—CO—) unterbrochen ist, steht, mit den Maßgaben, daß $R_1$ keine durch eine $C_{1-6}$Alkoxygruppe substituierte $C_{1-6}$-Alkylgruppe ist und daß, wenn die Alkylenkette durch ein Sauerstoff- oder Schwefelatom oder eine Sulfoxid-, Amid- (CONH) oder Ester- (COO)-Gruppe unterbrochen ist, mindestens zwei Kohlenstoffatome zwischen dieser Gruppe und dem Stickstoffatom, an das $R_1$ augefügt ist, vorhanden sein müssen,

$R_2$ für Wasserstoff oder eine $C_{1-4}$-Alkylgruppe steht,

Alk für eine gerade oder verzweigte Alkylenkette mit 1 bis 6 Kohlenstoffatomen steht,

Q für einen Furan- oder Thiophenring steht, dessen Einarbeitung in den Rest des Moleküls durch Bindungen in 2- und 5-Stellungen erfolgt ist, wobei der Furanring gegebenenfalls einen weiteren Substituenten $R_6$, angrenzend an die Gruppe $R_1R_2NAlk$-, trägt, oder Q für einen Benzolring steht, dessen Einarbeitung in den Rest des Moleküls durch Bindungen in 1- und 3- oder 1- und 4-Stellungen erfolgt ist,

$R_6$ für Halogen oder $C_{1-4}$-Alkyl, das durch Hydroxy oder $C_{1-4}$-Alkoxy substituiert sein kann, steht,

X für —$CH_2$—, —O—, —S— oder —NH— steht,

n den Wert 0, 1 oder 2 hat,

m den Wert 2, 3 oder 4 hat,

$R_3$ für Wasserstoff, $C_{1-6}$-Alkyl, $C_{3-6}$-Alkenyl oder $C_{2-6}$-Alkyl, das durch Hydroxy oder $C_{1-6}$-Alkoxy substituiert ist, steht und

$R_4$ für Wasserstoff, $C_{1-6}$-Alkyl, $C_{3-6}$-Alkenyl, Phenyl-$C_{1-3}$-alkyl, Hydroxy-$C_{1-6}$-alkyl, $C_{1-6}$-Alkanoyloxy-$C_{1-6}$-alkyl, $C_{1-6}$-Alkoxy-$C_{1-6}$-alkyl, Amino-$C_{1-6}$-alkyl, $C_{1-6}$-Alkylamino-$C_{1-6}$-alkyl. Di-$C_{1-6}$-alkylamino-$C_{1-6}$-alkyl, Hydroxy oder $C_{1-6}$-Alkoxy oder die Gruppe $NR_7R_8$ steht, wobei

$R_7$ für Wasserstoff, $C_{1-6}$-Alkyl, $C_{1-6}$-Alkyl, substituiert durch Hydroxy oder $C_{1-3}$-Alkoxy, $C_{3-6}$-Alkenyl, Phenyl-$C_{1-3}$-alkyl oder Heteroaryl-$C_{1-6}$-alkyl steht und $R_8$ für eine der für $R_7$ definierten Gruppen steht oder die Gruppe $COR_9$ darstellen kann, wobei $R_9$ für Wasserstoff, $C_{1-6}$-Alkyl, Phenyl, gegebenenfalls substituiert durch eine $C_{1-3}$-Alkyl oder $C_{1-3}$-Alkoxygruppe, $C_{1-6}$-Alkoxy, Heteroaryl oder Heteroaryl-$C_{1-6}$-alkyl steht, oder $R_8$ für die Gruppe $SO_2R_{10}$ steht, wobei $R_{10}$ für $C_{1-6}$-Alkyl oder Phenyl, gegebenenfalls substituiert durch eine $C_{1-3}$-Alkyl- oder $C_{1-3}$-Alkoxygruppe, steht oder $R_8$ für die Gruppe

$$\underset{\underset{\displaystyle Y}{\|}}{C}\text{—}NHR_{11}$$

steht, wobei

Y für Sauerstoff oder Schwefel steht und $R_{11}$ für Wasserstoff, $C_{1-6}$-Alkyl, $C_{3-8}$-Cycloalkyl oder Phenyl, gegebenenfalls substituiert durch eine $C_{1-3}$-Alkyl- oder $C_{1-3}$-Alkoxygruppe, steht oder $R_7$ und $R_8$, zusammengenommen, die Gruppe $=CR_{12}R_{13}$ darstellen, wobei $R_{12}$ für phenyl oder Heteroaryl steht und $R_{13}$ für Wasserstoff oder $C_{1-6}$-Alkyl steht, mit den Maßgaben, daß

(1) wenn X für —NH— steht, dann n den Wert 0 hat, und
(2) daß in der Definition der Gruppe $R_4$ die Bezeichnung "Heteroaryl" Furyl, Pyridyl, Thiazolyl oder Thienyl bedeutet.

2. Verbindungen nach Anspruch 1, dadurch gekennzeichnet, daß die Gruppen $R_1$, $R_2$, $R_3$ und $R_4$ die folgenden Bedeutungen haben:

$R_1$: eine Heteroalkylgruppe, deren heterocyclischer Teil ein Furyl-, Thienyl-, Pyrrolyl-, Pyridinyl-, Pyrimidinyl-, Triazinyl-, Oxazolyl-, Triazolyl-, Thiazolyl-, Isochinolinyl-, Chinolinyl- oder Indolylring ist, welcher Ring unsubstituiert ist oder durch einen Substituenten, ausgewählt aus $C_{1-3}$-Alkyl, $C_{1-3}$-Alkoxy, Hydroxy, Hydroxy-$C_{1-4}$-alkyl, Amino-$C_{1-4}$-alkyl, $C_{1-4}$-Alkylamino-$C_{1-4}$-alkyl, Di-$C_{1-4}$-alkylamino-$C_{1-4}$-alkyl und Halogen, substituiert ist, und deren Alkylenteil eine Methylen-, Ethylen- oder Propylengruppierung oder eine Alkylenkette, die gesättigt ist und bis zu 16 Kohlenstoffatome enthält oder die durch eine Amid- oder Estergruppierung substituiert oder unterbrochen ist, ist,

$R_2$: Wasserstoff oder eine Methyl- oder Ethylgruppe,

$R_3$: Wasserstoff, $C_{1-4}$-Alkyl oder Hydroxy-$C_{2-4}$-alkyl,

$R_4$: Wasserstoff, Hydroxy, $C_{1-4}$-Alkyl, Hydroxy-$C_{1-4}$-alkyl, $C_{1-3}$-Alkoxy-$C_{1-4}$-alkyl, Phenyl-$C_{1-3}$-alkyl, $C_{2-4}$-Alkenoyloxy-$C_{1-4}$-alkyl, Amino-$C_{1-4}$-alkyl, Amino, $C_{1-4}$-Alkylamino oder Di-$C_{1-4}$-alkylamino, Phenyl-$C_{1-3}$-alkylamino oder eine Heteroaryl-$C_{1-3}$-alkylaminogruppe, deren Heteroarylring Furyl, Pyridyl, Thiazolyl oder Thienyl ist, oder die Gruppe $NHCOR_9$, worin $R_9$ für Wasserstoff, $C_{1-3}$-Alkyl, $C_{1-3}$-Alkoxy, Furyl, Pyridyl, Thiazolyl, Thienyl oder Phenyl, gegebenenfalls durch eine $C_{1-3}$-Alkyl-

oder $C_{1-3}$-Alkoxygruppe substituiert, steht, oder die Gruppe $NHSO_2R_{10}$, worin $R_{10}$ für $C_{1-3}$-Alkyl oder Phenyl, das gegebenenfalls durch eine $C_{1-3}$-Alkyl- oder $C_{1-3}$-Alkoxygruppe substituiert ist, steht, oder die Gruppe $NHCONHR_{11}$, worin $R_{11}$ für $C_{1-3}$-Alkyl, $C_{5-7}$-Cycloalkyl oder Phenyl, das gegebenenfalls durch eine $C_{1-3}$-Alkyl- oder $C_{1-3}$-Alkoxygruppe substituiert ist, steht, oder die Gruppe $N{=}CHR_{12}$, worin $R_{12}$ für eine Phenyl- oder Pyridylgruppe steht,

die Gruppe Alk $(CH_2)_p$ ist, wobei p den Wert 1, 2 oder 3 hat, und die Gruppe Q ein Benzolring ist, der in den Rest des Moleküls durch Bindungen in 1- und 3- oder 1- und 4-Stellungen eingearbeitet ist, mit n als 0, X als Sauerstoff and m als 3 oder 4 oder die Gruppe Q ein Furan- oder Thiophenring ist, mit n als 1, X als Schwefel und m als 2, wobei der Furanring gegebenenfalls einen weiteren Substituenten $R_6$ trägt, wobei $R_6$ für Brom, $C_{1-3}$-Alkyl oder $C_{1-3}$-Alkoxy-$C_{1-3}$-alkyl steht, oder die Gruppe Q ein Furanring mit n als 1, X als $CH_2$ und m als 2 ist.

3. Verbindungen nach Anspruch 2, dadurch gekennzeichnet, daß die Gruppen $R_1$, $R_2$, $R_3$ unf $R_4$ die folgenden Bedeutungen haben:

$R_1$: eine Heteroarylmethylgruppe, wobei der Heteroarylring Furyl, Thienyl, Pyrrolyl oder Pyridinyl ist, das mit dem Methylteil über ein Kohlenstoffatom verbunden ist, oder eine gerade oder verzweigte gesättigte $C_{11-16}$-Alkylenkette oder eine gerade oder verzweigte gesättigte $C_{1-16}$-Alkylenkette, die durch eine Amid (—NHCO—)-Gruppe unterbrochen ist,
$R_2$: Wasserstoff oder eine Methyl- oder Ethylgruppe,
$R_3$: Wasserstoff, Methyl, Ethyl oder 2-Hydroxyethyl,
$R_4$: Hydroxy, Phenyl-$C_{1-3}$-alkyl, $C_{1-4}$-Alkyl, substituiert durch Hydroxy, $C_{1-3}$-Alkoxy, $C_{2-4}$-Alkenoyloxy oder Amino, Amino, Di-$C_{1-4}$-alkylamino, $NHCOR_9$, wobei $R_9$ für Wasserstoff, $C_{1-3}$-Alkoxy oder Phenyl steht, $NHCONHR_{11}$, wobei $R_{11}$ für Phenyl steht, oder $N{=}CHR_{12}$, wobei $R_{12}$ für Phenyl oder Pyridyl steht,

die Gruppe Alk eine Methylen- oder Ethylengruppe ist und die Gruppe Q ein Benzolring ist, der in den Rest des Moleküls durch Bindungen in 1- und 3- oder 1- und 4-Stellungen eingefügt ist, mit n als 0, X als Sauerstoff und m als 3 oder 4, oder die Gruppe Q ein Furan- oder Thiophenring ist, mit n als 1, X als Schwefel und m als 2, wobei der Furanring gegebenenfalls einen weiteren Substituenten $R_6$ enthält, wobei $R_6$ für Brom, $C_{1-3}$-Alkyl oder $C_{1-3}$-Alkoxy-$C_{1-3}$-alkyl steht, oder die Gruppe Q ein Furanring ist mit n als 1, X als $CH_2$ und m als 2.

4. Verbindungen nach einem der Ansprüche 1 bis 3, dadurch gekennzeichnet, daß die Gruppe Q ein Benzolring ist, der in den Rest des Moleküls durch Bindungen in 1- und 3-Stellungen eingearbeitet ist, mit n als 0, X als Sauerstoff und m als 3 oder 4, oder die Gruppe Q ein Furanring ist, der gegebenenfalls einen weiteren Substituenten $R_6$, trägt, wobei $R_6$ für $C_{1-4}$-Alkyl steht, mit n als 1, X als Schwefel und m als 2.

5. Verbindungen nach Anspurch 1 oder 2, dadurch gekennzeichnet, daß $R_1$ eine Heteroaralkylgruppe ist, die aus einem heterocyclischen Teil, nämlich einem Furyl-, Thienyl-, Pyrrolyl-, Pyridinyl-, Pyrimidinyl-, Triazinyl-, Oxazolyl-, Triazolyl-, Thiazolyl-, Isochinolinyl-, Chinolinyl- oder Indolylring, welcher Ring unsubstituiert ist, oder durch einen Substituenten, ausgewählt aus $C_{1-3}$-Alkyl, $C_{1-3}$-Alkoxy, Hydroxy, Hydroxy-$C_{1-4}$-alkyl, Amino-$C_{1-4}$-alkyl, $C_{1-4}$-Alkylamino-$C_{1-4}$-alkyl, Di-$C_{1-4}$-alkylamino-$C_{1-4}$-alkyl und Halogen, substituiert ist, und welcher Ring mit dem Alkylteil durch Kohlenstoff verbunden ist, und einem Alkylteil, nämlich einer geraden oder verzweigten $C_{1-4}$-Alkylkette, besteht.

6. Verbindungen nach Anspruch 5, dadurch gekennzeichnet, daß sie der Formel (II):

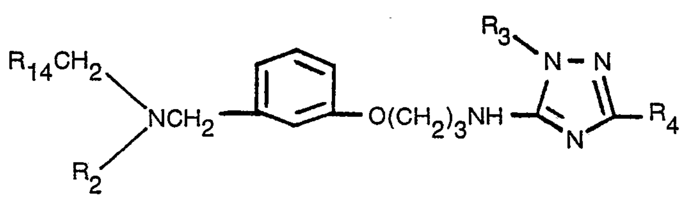

(II)

entsprechen, wobei $R_{14}$ ein Furyl-, Thienyl-, Pyrrolyl- oder Pyridinylring ist, welcher durch ein Kohlenstoffatom angeknüpft ist, $R_2$ für Wasserstoff oder Methyl steht, $R_3$ für Wasserstoff oder Methyl steht und $R_4$ eine Amino-, Hydroxy-$C_{1-6}$-alkyl-, $C_{1-6}$-Alkanoyloxy-$C_{1-6}$-alkyl-, $C_{1-6}$-Alkoxy-$C_{1-6}$-alkyl-, Amino-$C_{1-6}$-alkyl-, Benzyl-, Formamido-, $C_{1-6}$-Alkoxycarbonylamino-, $C_{2-7}$-Alkanoylamino-, Hydroxy-, Aroylamino- (wobei die Arylgruppe Phenyl ist, das gegebenenfalls durch eine $C_{1-3}$-Alkyl- oder $C_{1-3}$-Alkoxygruppe substituiert ist). Phenylcarbamoylamino- oder Pyridylmethylenaminogruppe ist.

7. Verbindungen nach Anspruch 6, dadurch gekennzeichnet, daß $R_4$ für Amino, Hydroxy-$C_{1-6}$-alkyl oder Pyridylmethylenamino steht.

8. 5-[[2-[[[5-[[N-Methyl-(2-furanylmethyl)-amino]-methyl]-2-furanyl]-methyl]-thio]-ethyl]-amino]-1-methyl-1H-1,2,4-triazol-3-methanol und seine physiologisch annehmbaren Salze.

9. 1-Methyl-$N^5$-[3-[3-[(dodecylamino)-methyl]-phenoxy]-propyl]-1H-1,2,4-triazol-3,5-diamin und seine physiologisch annehmbaren Salze.

10. 1-Methyl-$N^5$-[3-[3-[(2-furanylmethyl)-aminomethyl]-phenoxy]-propyl]-1H-1,2,4-triazol-3,5-diamin, 1-Methyl-$N^5$-[3-[3-[(1H-pyrrol-2-yl-methyl)-aminomethyl]-phenoxy]-propyl]-1H-1,2,4-triazol-3,5-diamin und ihre physiologisch annehmbaren Salze.

11. Verbindungen nach Anspruch 1, dadurch gekennzeichnet, daß $R_1$ für eine Heteroaralkylgruppe steht, die aus einem heterocyclischen Teil, nämlich einem Furyl-, Thienyl-, Pyrrolyl-, Pyridinyl-, Pyrimidinyl-, Triazinyl-, Oxazolyl-, Triazolyl-, Thiazolyl-, Isochinolinyl-, Chinolinyl- oder Indolylring, welcher Ring unsubstituiert ist oder durch einen Substituenten, ausgewählt aus $C_{1-3}$-Alkyl, $C_{1-3}$-Alkoxy, Hydroxy, Hydroxy-$C_{1-4}$-alkyl, Amino-$C_{1-4}$-alkyl, $C_{1-4}$-Alkylamino-$C_{1-4}$-alkyl, Di-$C_{1-4}$-alkylamino-$C_{1-4}$-alkyl und Halogen, substituiert ist und welcher Ring mit dem Alkylteil durch Kohlenstoff verbunden ist, und einem Alkylteil, nämlich einer geraden oder verzweigten $C_{1-4}$-Alkylkette, besteht und daß

$R_4$ für $NR_7R_8$ steht, wobei $R_7$ und $R_8$ jeweils für Wasserstoff, $C_{1-6}$-Alkyl, $C_{1-6}$-Alkyl, substituiert durch Hydroxy oder $C_{1-3}$-Alkoxy, $C_{3-6}$-Alkenyl, Phenyl-$C_{1-3}$-alkyl oder Heteroaryl-$C_{1-6}$-alkyl stehen oder $R_7$ und $R_8$ miteinander $=CR_{12}R_{13}$ darstellen, wobei $R_{12}$ für Phenyl oder Heteroaryl steht und $R_{13}$ für Wasserstoff oder $C_{1-6}$-Alkyl steht (wobei Heteroaryl innerhalb der Definition von $R_4$ Furyl, Pyridyl, Thiazolyl oder Thienyl bedeutet).

12. Verfahren zur Herstellung von Verbindungen der Formel (I), wie im Anspruch 1 definiert, dadurch gekennzeichnet, daß man

(a) zur Herstellung von Verbindungen, bei denen Alk für $CH_2$ steht und $R_4$ eine andere Bedeutung als $—N{=}CR_{12}R_{13}$ hat, eine Verbindung der Formel (III):

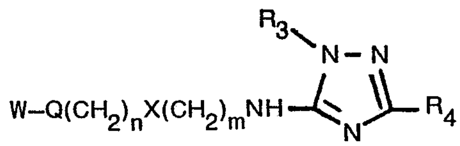

(III)

worin W für die Gruppe —CHO steht, mit einem Reagenz der Formel $R_1R_2NH$ umsetzt und anschließend das so gebildete Zwischenprodukt mit einem Hydrid-Reduktionsmittel oder Wasserstoff und einem Metallkatalysator reduziert,

(b) zur Herstellung von Verbindungen, bei denen $R_4$ eine andere Bedeutung als $—N{=}CR_{12}R_{13}$ hat, eine Verbindung der Formel (IV):

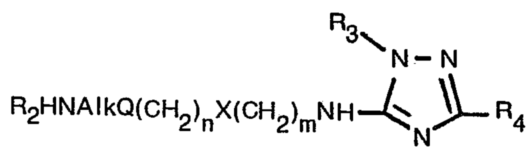

(IV)

mit einem Aldehyd $R_1^1CHO$ unter Reduktionsbedingungen, die durch ein Hydrid-Reduktionsmittel oder Wasserstoff und einen Metallkatalysator zur Verfügung gestellt werden, umsetzt, wobei die Gruppe $R_1^1$ eine derartige Bedeutung hat, daß die resultierende Gruppe $R_1^1CH_2—$ eine Gruppe $R_1$ ist,

(c) eine Verbindung der Formel (IV), wie oben definiert, mit einer Verbindung $R_1Hal$ umsetzt, wobei Hal für Halogen steht,

(d) zur Herstellung von Verbindungen, bei denen $R_4$ die Bedeutung $N{=}CR_{12}R_{13}$ hat, eine Verbindung der Formel (I), in der $R_4$ die Bedeutung $NH_2$ hat, mit einem Aldehyd oder Keton $R_{12}R_{13}CO$ umsetzt,

(e) zur Herstellung von Verbindungen, bei denen $R_4$ eine Acyloxyalkylgruppe ist, eine Verbindung, bei der $R_4$ die Bedeutung Hydroxyalkyl hat, mit einer geeigneten Säure umsetzt,

(f) zur Herstellung von Verbindungen, bei denen $R_4$ die Gruppe $NR_7R_8$ ist, wobei $R_8$ für $—COR_9$, $—SO_2R_{10}$ oder $—C({=}Y)NHR_{11}$ steht, ein Aminotriazol (V):

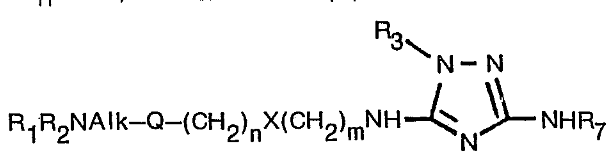

(V)

worin $R_1$, $R_2$, $R_3$ und $R_7$ wie im Zusammenhang mit der Formel (I) definiert sind oder Gruppen darstellen, die leicht in solche Gruppen umwandelbar sind, mit

(i) einem aktivierten Derivat einer Carbonsäure $R_9COOH$ oder einer Sulfonsäure $R_{10}SO_3H$ zum Austausch des Wasserstoffatoms in der Gruppe $NHR_7$ durch $—COR_9$ oder $—SO_2R_{10}$, oder

(ii) einem Isocyanat oder Isothiocyanat $R'_{11}NCY$, wobei $R'_{11}$ eine der für $R_{11}$ in der Formel (I) angegebene Bedeutung, ausgenommen Wasserstoff, hat, oder ein Alkalimetallatom oder eine Alkoxycarbonylgruppe ist, zum Austausch des Wasserstoffatoms in der Gruppe $NHR_7$ durch $—C({=}Y)NHR_{11}$, behandelt,

(g) zur Herstellung von Verbindungen, bei denen $R_1$ eine Alkylenkette ist, die durch eine Amidgruppierung unterbrochen ist, eine Verbindung der Formel (I), worin $R_1$ eine Alkylenkette, die durch

eine Estergruppierung $CO_2R_5$ substituiert ist, ist (d.h. $R_1$ die Gruppe $R_5OOC(CH_2)_x$—) ist), mit einem Amin $CH_3(CH_2)_yNH_2$ behandelt, um eine Verbindung der Formel (I) zu erhalten, bei der $R_1$ die Gruppe $CH_3(CH_2)_yNHCO(CH_2)_x$— ist, wobei x und y ganze Zahlen sind und die Summe von x und y nicht größer als 15 ist,

und wenn die Verbindung der Formel (I) in Form einer freien Base erzeugt wird, gegebenenfalls die freie Base in ein Salz umwandelt.

13. Arzneimittel, dadurch gekennzeichnet, daß es eine Verbindung nach den Ansprüchen 1 bis 11 zusammen mit mindestens einem pharmazeutisch annehmbaren Träger oder Verdünnungsmittel und gegebenenfalls einem oder weiteren Wirkstoffen enthält.

**Revendications**

1. Composés de formule générale (I)

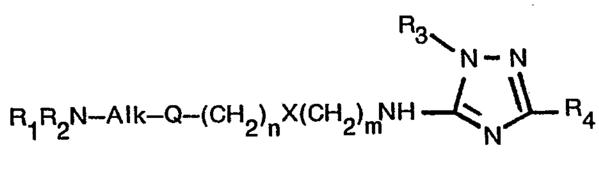

(I)

et sels et hydrates phyiologiquement acceptables de ceux-ci, formule dans laquelle

$R_1$ représente un groupe hétéroaralkyle constitué d'une partie hétérocyclique qui est un noyau furyle, thiényle, pyrrolyle, pyridinyle, pyrimidinyle, triazinyle, oxazolyle, triazolyle, thiazolyle, isoquinoléinyle, quinoléinyle ou indolyle, ce noyau étant non substitué ou substitué par un substituant choisi parmi les groupes alkyle en $C_{1-3}$, alcoxy en $C_{1-3}$, hydroxy, hydroxyalkyle en $C_{1-4}$, aminoalkyle en $C_{1-4}$, alkyl ($C_{1-4}$) amino-alkyle en $C_{1-4}$, dialkyl ($C_{1-4}$) amino-alkyle en $C_{1-4}$ et halogène, ce noyau étant lié au fragment alkyle par l'intermédiaire d'un carbone, et le fragment alkyle étant une chaîne alkyle droite ou ramifiée en $C_{1-4}$,

ou bien $R_1$ représente une chaîne alkylène saturée ou insaturée, droite ou ramifiée en $C_{11-16}$, facultativement substituée par un groupe hydroxy, ou bien $R_1$ représente une chaîne alkylène saturée ou insaturée, droite ou ramifiée en $C_{1-16}$ substituée par un groupe ester $CO_2R_5$ ou un groupe amide $CONH_2$, dans lequel $R_5$ est un radical alkyle en $C_{1-3}$, ou bien $R_1$ représente une chaîne alkylène saturée ou insaturée, droite ou ramifiée en $C_{1-16}$ interrompue par un atome d'oxygène, un atome de soufre, un groupe sulfoxyde, un groupe sulfone, un groupe amide (—COHN— ou —NHCO—) ou un groupe ester (—CO—O— ou —O—CO—), aux conditions que

$R_1$ ne représente pas un radical alkyle en $C_{1-6}$ substitué par un radical alcoxy en $C_{1-6}$ et que lorsque la chaîne alkylène est interrompue par un atome d'oxygène ou de soufre ou un sulfoxyde, un amide (CONH) ou un groupe ester (COO), au moins deux atomes de carbone dussent être présente entre ce groupe et l'atome d'azote auquel $R_1$ est fixé;

$R_2$ représente un atome d'hydrogène ou un radical alkyle en $C_{1-4}$;

Alk représente une chaîne alkylène droite ou ramifiée de 1 à 6 atomes de carbone;

Q représente un noyau furanne ou thiophène dans lequel l'incorporation dans le reste de la molécule se fait à travers des liaisons dans les positions 2 et 5, le noyau furanne portant facultativement un autre substituant $R_6$ adjacent au groupe $R_1R_2NAlk$- ou bien Q représente un noyau benzénique dans lequel l'incorporation dans le reste de la molécule se fait à travers des liaisons dans les positions 1 et 3 ou 1 et 4;

$R_6$ représente un atome d'halogène ou un radical alkyle en $C_{1-4}$, qui peut être substitué par un groupe hydroxy ou alcoxy en $C_{1-4}$;

X représente —$CH_2$—, —O—, —S— ou —NH—;

*n* représente zéro, 1 ou 2;

*m* représente 2, 3 ou 4;

$R_3$ représente un atome d'hydrogène ou un radical alkyle en $C_{1-6}$, alcényle en $C_{3-6}$, ou alkyle en $C_{2-6}$ substitué par hydroxy ou alcoxy en $C_{1-6}$; et

$R_4$ représente un atome d'hydrogène ou un radical alkyle en $C_{1-6}$, alcényle en $C_{3-6}$, phénylalkyle en $C_{1-3}$, hydroxyalkyle en $C_{1-6}$, alcanoyloxy ($C_{1-6}$) alkyle en $C_{1-6}$, alcoxy ($C_{1-6}$) alkyle en $C_{1-6}$, aminoalkyle en $C_{1-6}$, alkyl ($C_{1-6}$) aminoalkyle en $C_{1-6}$, dialkyl ($C_{1-6}$) aminoalkyle en $C_{1-6}$, hydroxy, alcoxy en $C_{1-6}$ ou le groupe $NR_7R_8$ dans lequel

$R_7$ représente un atome d'hydrogène, un radical alkyle en $C_{1-6}$, alkyle en $C_{1-6}$ substitué par hydroxy ou alcoxy en $C_{1-3}$, alcényle en $C_{3-6}$, phénylalkyle en $C_{1-3}$ ou hétéroarylalkyle en $C_{1-6}$ et $R_8$ représente l'un quelconque des groupes définis pour $R_7$ ou peut représenter le groupe $COR_9$ dans lequel $R_9$ est un atome d'hydrogène, un radical alkyle en $C_{1-6}$, phényle facultativement substitué par un groupe alkyle en $C_{1-3}$ ou alcoxy en $C_{1-3}$, alcoxy en $C_{1-6}$, hétéroaryle ou hétéroarylalkyle en $C_{1-6}$ ou bien $R_8$ représente le groupe $SO_2R_{10}$ dans lequel $R_{10}$ est un radical alkyle en $C_{1-6}$ ou phényle facultativement substitué par un radical alkyle en $C_{1-3}$ ou alcoxy en $C_{1-3}$, ou bien $R_8$ représente le groupe

$$\underset{\underset{Y}{\|}}{C}-NHR_{11}$$

dans lequel Y est l'oxygène ou le soufre et

$R_{11}$ représente un atome d'hydrogène, un radical alkyle en $C_{1-6}$, cycloalkyle en $C_{3-8}$ ou phényle facultativement substitué par un groupe alkyle en $C_{1-3}$ ou alcoxy en $C_{1-3}$,

ou bien $R_7$ et $R_8$ ensemble peuvent représenter le groupe $=CR_{12}R_{13}$ dans lequel $R_{12}$ est un radical phényle ou hétéroaryle et $R_{13}$ est un atome d'hydrogène ou un radical alkyle en $C_{1-6}$, aux conditions que (1) quand X est —NH—, alors *n* soit zéro et (2) dans la définition du groupe $R_4$, "hétéroaryle" désigne furyle, pyridyle, thiazolyle ou thiényle.

2. Composés selon la revendication 1 dans lesquels les groupes $R_1$, $R_2$, $R_3$ et $R_4$ ont les significations suivantes:

$R_1$: un groupe hétéroalkyle dans lequel la partie hétérocyclique est un noyau furyle, thiényle, pyrrolyle, pyridinyle, pyrimidinyle, triazinyle, oxazolyle, triazolyle, thiazolyle, isoquinoléinyle, quinoléinyle ou indolyle, ce noyau étant non substitué ou substitué par un substituant choisi parmi les radicaux alkyle en $C_{1-3}$, alcoxy en $C_{1-3}$, hydroxy, hydroxyalkyle en $C_{1-4}$, aminoalkyle en $C_{1-4}$, alkyl ($C_{1-4}$) aminoalkyle en $C_{1-4}$, dialkyl ($C_{1-4}$) aminoalkyle en $C_{1-4}$ et halogène, et la portion alkylène est un groupement méthylène, éthylène ou propylène; ou une chaîne alkylène qui est saturée et contient jusqu'à 16 atomes de carbone ou est substituée ou interrompue par un groupement amide ou ester;

$R_2$: un atome d'hydrogène ou un radical méthyle ou éthyle;

$R_3$: un atome d'hydrogène, ou un radical alkyle en $C_{1-4}$ ou hydroxyalkyle en $C_{2-4}$;

$R_4$: un atome d'hydrogène ou un radical hydroxy, alkyle en $C_{1-4}$, hydroxyalkyle en $C_{1-4}$, alcoxy ($C_{1-3}$) alkyle en $C_{1-4}$, phénylalkyle en $C_{1-3}$, alcanoyl ($C_{2-4}$) oxyalkyle en $C_{1-4}$, aminoalkyle en $C_{1-4}$, amino, alkylamino en $C_{1-4}$ ou dialkylamino en $C_{1-4}$, phénylalkylamino en $C_{1-3}$ ou un groupe hétéroaryl-alkylamino en $C_{1-3}$ dans lequel le noyau hétéroaryle est furyle, pyridyle, thiazolyle ou thiényle; ou bien le groupe $NHCOR_9$ dans lequel $R_9$ représente un atome d'hydrogène, un radical alkyle en $C_{1-3}$, alcoxy en $C_{1-3}$, furyle, pyridyle, thiazolyle, thiényle, ou phényle facultativement substitué par un radical alkyle en $C_{1-3}$ ou alcoxy en $C_{1-3}$; ou le groupe $NHSO_2R_{10}$ dans lequel $R_{10}$ représente un radical alkyle en $C_{1-3}$ ou phényle facultativement substitué par un radical alkyle en $C_{1-3}$ ou alcoxy en $C_{1-3}$; ou le groupe $NHCONHR_{11}$ dans lequel $R_{11}$ est un radical alkyle en $C_{1-3}$, cycloalkyle en $C_{5-7}$ ou phényle facultativement substitué par un radical alkyle en $C_{1-3}$ ou alcoxy en $C_{1-3}$; ou le groupe $N{=}CHR_{12}$ dans lequel $R_{12}$ est un radical phényle ou pyridyle;

le groupe Alk représente $(CH_2)_p$ dans lequel *p* est 1, 2 ou 3; et le groupe Q est un noyau de benzène incorporé dans le reste de la molécule à travers des liaisons dans les positions 1- et 3- ou 1- et 4-, *n* étant zéro, X étant un atome d'oxygène et *m* étant 3 ou 4; ou bien le groupe Q est un noyau furannique ou de thiophène, *n* étant 1, X étant un atome de soufre et *m* étant 2, le noyau furannique contenant facultativement un autre substituant $R_6$ qui est un atome de brome, un radical alkyle en $C_{1-3}$, alcoxy ($C_{1-3}$) alkyle $C_{1-3}$; ou le groupe Q est un noyau furannique, *n* étant 1, X étant $CH_2$ et *m* étant 2.

3. Composés selon la revendication 2, dans lesquels les groupes $R_1$, $R_2$, $R_3$ et $R_4$ ont les significations suivantes:

$R_1$: un groupe hétéroarylméthyle dans lequel le noyau hétéroaryle est furyle, thiényle, pyrrolyle ou pyridinyle lié à la portion méthylique à travers un atome de carbone; ou bien une chaîne alkylène saturée linéaire ou ramifiée en $C_{11-16}$; ou une chaîne alkylène saturée droite ou ramifiée en $C_{1-16}$ interrompue par un groupe amide (liaison NHCO);

$R_2$: un atome d'hydrogène ou un radical méthyle ou éthyle;

$R_3$: un atome d'hydrogène ou un radical méthyle, éthyle, ou 2-hydroxyéthyle;

$R_4$: un groupe hydroxy, phénylalkyle en $C_{1-3}$, alkyle en $C_{1-4}$ substitué par un groupe hydroxy, alcoxy en $C_{1-3}$, alcanoyloxy en $C_{2-4}$ ou amino; amino, dialkylamino en $C_{1-4}$, $NHCOR_9$ dans lequel $R_9$ représente un atome d'hydrogène, un radical alcoxy en $C_{1-3}$ ou phényle, $NHCONHF_{11}$ dans lequel $R_{11}$ représente un radical phényle, ou $N{=}CHR_{12}$ dans lequel $R_{12}$ est le radial phényle ou pyridyle; le groupe Alk est un groupe méthylène ou éthylène; et

le groupe Q est un noyau de benzène incorporé dans le reste de la molécule à travers les liaisons dans les positions 1 et 3 ou 1 et 4, *n* étant zéro, X étant un atome d'oxygène et *m* étant 3 ou 4; ou le groupe Q est un noyau de furanne ou de thiophène, *n* étant 1, X étant un atome de soufre, et *m* étant 2, le noyau de furanne contenant facultativement un autre substituant $R_6$ qui est un atome de brome, un radical alkyle en $C_{1-3}$ ou un radical alcoxy ($C_{1-3}$) alkyle $C_{1-3}$; ou bien le groupe Q est un noyau de furanne quand *n* est 1, X est $CH_2$ et *m* est 2.

4. Composés selon l'une quelconque des revendications 1 à 3, dans lesquels le groupe Q est un noyau de benzène incorporé dans le reste de la molécule à travers les liaisons dans les positions 1 et 3,

*n* étant zéro, X étant un atome d'oxygène et *m* étant 3 ou 4; ou bien le groupe Q est un noyau de furanne contenant facultativement un autre substituant $R_6$ qui est un radical alkyle en $C_{1-4}$, alors que *n* est 1, X est un atome de soufre et *m* est 2.

5. Composés selon la revendication 1 ou 2, dans lesquels $R_1$ est un groupe hétéroaralkylique constitué d'une partie hétérocyclique qui est un noyau de furyle, thiényle, pyrrolyle, pyridinyle, pyrimidinyle, triazinyle, oxazolyle, triazolyle, thiazolyle, isoquinoléinyle, quinoléinyle ou indolyle, ce noyau étant non substitué ou substitué par un substituant choisi parmi les radicaux alkyle en $C_{1-3}$, alcoxy en $C_{1-3}$, hydroxy, hydroxyalkyle en $C_{1-4}$, aminoalkyle en $C_{1-4}$, alkylamino $(C_{1-4})$ alkyle $C_{1-4}$, dialkyl $(C_{1-4})$ aminoalkyle en $C_{1-4}$ et halogène, ce noyau étant lié à la partie alkylique à travers un carbone, et une partie alkylique qui est une chaîne alkyle en $C_{1-4}$ droite ou ramifiée.

6. Composés selon la revendication 5 qui correspondent à la formule (II):

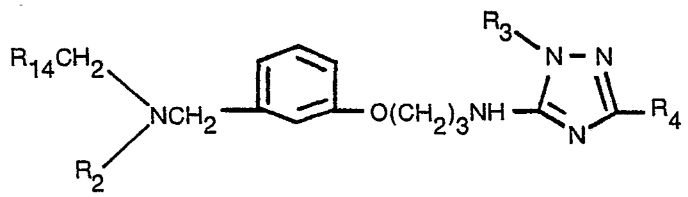
(II)

dans laquelle $R_{14}$ est un noyau furyle, thiényle, pyrrolyle ou pyridinyle lié à travers un atome de carbone, $R_2$ est un atome d'hydrogène ou le radical méthyle; $R_3$ est un atome d'hydrogène ou un radical méthyle; et $R_4$ est un radical amino, hydroxyalkyle en $C_{1-6}$, alcanoyl $(C_{1-6})$ oxyalkyle en $C_{1-6}$, alcoxy $(C_{1-6})$ alkyle en $C_{1-6}$, aminoalkyle en $C_{1-6}$, benzyle, formamido, alcoxycarbonylamino en $C_{1-6}$, alcanoylamino en $C_{2-7}$, hydroxy, aroylamino (dans lequel le radical aryle est le phényle facultativement substitué par un groupe alkyle en $C_{1-3}$ ou alcoxy en $C_{1-3}$), phénylcarbamoylamino ou pyridylméthylèneamino.

7. Composés selon la revendication 6, dans lesquels $R_4$ représente un groupe amino, hydroxyalkyle en $C_{1-6}$ ou pyridylméthylène-amino.

8. 5-[[2-[[[5-[[N-méthyl-(2-furannylméthyl)amino]méthyl]-2-furannyl]méthyl]thio]éthyl]amino]-1-méthyl-1H-1,2,4-triazole-3-méthanol et ses sels physiologiquement acceptables.

9. 1-méthyl-$N^5$-[3-[3-[(dodécylamino)méthyl]phénoxy]propyl]-1H-1,2,4-triazole-3,5-diamine et ses sels physiologiquement acceptables.

10. 1-méthyl-$N^5$-[3-[3-[(2-furanylméthyl)aminométhyl]phénoxy]propyl]-1H-1,2,4-triazole-3,5-diamine-1-méthyl-$N^5$-[3-[3-[(1H-pyrrol-2-yl-méthyl)aminométhyl]phénoxy]propyl]1H-1,2,4-triazole-3,5-diamine et leurs sels physiologiquement acceptables.

11. Composés selon la revendication 1, dans lesquels

$R_1$ représente un groupe hétéroaralkyle constitué d'une partie hétérocyclique qui est un noyau furyle, thiényle, pyrrolyle, pyridinyle, pyrimidinyle, triazinyle, oxazolyle, triazolyle, thiazolyle, isoquinoléinyle, quinoléinyle ou indolyle, ce noyau étant non substitué ou substitué par un substituant choisi parmi les radicaux alkyle en $C_{1-3}$, alcoxy en $C_{1-3}$, hydroxy, hydroxyalkyle en $C_{1-4}$, aminoalkyle en $C_{1-4}$, alkyl $(C_{1-4})$ aminoalkyle en $C_{1-4}$, dialkyl $(C_{1-4})$ aminoalkyle en $C_{1-4}$ et halogène, ce noyau étant lié à la partie alkylique par un carbone, et une partie alkylique qui est une chaîne alkyle droite ou ramifiée en $C_{1-4}$; et

$R_4$ représente $NR_7R_8$ dans lequel $R_7$ et $R_8$ représentent chacun un atome d'hydrogène, un radical alkyle en $C_{1-6}$, un radical alkyle en $C_{1-6}$ substitué par un groupe hydroxy un alcoxy en $C_{1-3}$, alcényle en $C_{3-6}$, phénylalkyle en $C_{1-3}$ ou hétéroarylalkyle en $C_{1-6}$, ou bien $R_7$ et $R_8$ représentent ensemble le groupe $=CR_{12}R_{13}$ dans lequel $R_{12}$ représente un radical phényle ou hétéroaryle et $R_{13}$ représente un atome d'hydrogène ou un radical alkyle en $C_{1-6}$ (où hétéroaryle en dedans de la définition ou $R_4$ signifie furyle, pyridyle, thiazolyle ou thiényle).

12. Procédé de production de composés de formule (I) selon la définition dans la revendication 1, qui consiste:

(a) pour la production de composés dans lesquels Alk est $CH_2$ et $R_4$ est autre que $—N{=}CR_{12}R_{13}$. A faire réagir un composé de formule (III)

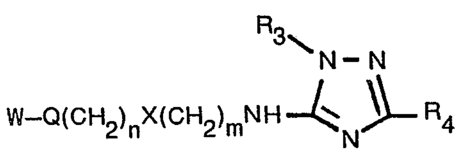
(III)

dans laquelle W représente le groupe —CHO avec un réactif de formule $R_1R_2NH$, puis à réduire le produit intermédiaire ainsi formé avec un agent réducteur d'hydrure ou l'hydrogène et un catalyseur métallique;

(b) pour la production de composés dans lesquels $R_4$ est autre que $—N{=}CR_{12}R_{13}$, à faire réagir un composé de formule (IV)

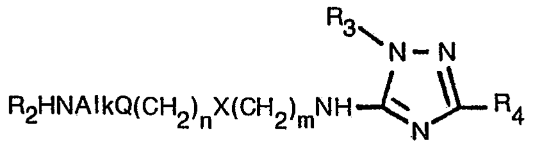 (IV)

avec un aldéhyde $R_1^1CHO$ dans des conditions réductrices fournies par un agent réducteur d'hydrure ou l'hydrogène et un catalyseur métallique, le groupe $R_1^1$ ayant une signification telle que le groupe résultant $R_1^1CH_2$— représente un groupe R;

(c) à faire réagir un composé de formule (IV) tel que défini plus haut avec un composé $R_1Hal$ dans lequel Hal est un halogène;

(d) pour la production de composés dans lesquels $R_4$ représente $N{=}CR_{12}R_{13}$, à faire réagir un composé de formule (I) dans lequel $R_4$ est $NH_2$ avec un aldéhyde ou une cétone $R_{12}R_{13}CO$;

(e) pour la production de composés dans lesquels $R_4$ représente un groupe acyloxyalkyle, à faire réagir un composé dans lequel $R_4$ représente un radical hydroxyalkyle avec un acide approprié;

(f) pour la production de composés dans lesquels $R_4$ représente le groupe $NR_7R_8$ dans lequel $R_8$ est $—COR_9$, $—SO_2R_{10}$ ou $—C(=Y)NHR_{11}$, à traiter un aminotriazole (V)

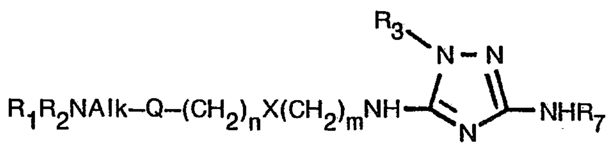 (V)

dans lequel $R_1$, $R_2$, $R_3$ et $R_7$ sont tels que définis dans la formule (I) ou sont des groupes facilement convertibles en ces derniers, avec

(i) un dérivé activé d'un acide carboxylique $R_9COOH$ ou d'un acide sulfonique $R_{10}SO_3H$ pour remplacer l'atome d'hydrogène dans le groupe $NHR_7$ par $—COR_9$ ou $—SO_2R_{10}$; ou

(ii) un isocyanate ou isothiocyanate $R'_{11}NCY$ dans lequel $R'_{11}$ a l'une des signification définies pour $R_{11}$ dans la formule (I) excepté l'hydrogène, ou bien 11 représente un atome de métal alcalin ou un groupe alcoxycarbonyle pour remplacer l'atome d'hydrogène dans le groupe $NHR_7$ par $—C(=Y)NHR_{11}$;

(g) pour la production des composés dans lesquels $R_1$ est une chaîne alkylène interrompue par un groupement amide, à traiter un composé de formule (I) dans lequel $R_1$ est une chaîne alkylène substituée par un groupement ester $CO_2R_5$ (c'est à dire que $R_1$ est le groupe $R_5OOC\,(CH_2)_x—$) avec une amine $CH_2(CH_2)_yNH_2$ pour donner un composé de formule (I) dans lequel $R_1$ représente le groupe $CH_3(CH_2)_yNHCO(CH_2)_x—$ dans lequel $x$ et $y$ sont des nombres entiers et le total de $x$ et $y$ ne dépasse pas 15;

et lorsque le composé de formule (I) est produit sous forme d'une base libre, à convertir facultativement la base libre en un sel.

13. Composition pharmaceutique comprenant un composé selon les revendications 1 à 11 ensemble avec au moins un véhicule ou diluant pharmaceutiquement acceptable, et facultativement un ou plusieurs autres ingrédients actifs.